# EUROPEAN PATENT APPLICATION

(11) **EP 4 775 209 A1**
(43) Date of publication of application: **15.07.2026**
(21) Application number: 24862034.6
(22) Date of filing: 05.09.2024
(51) Int. Cl.: A61K 31/664, A61P 35/00, A61P 35/02

(54) **ADMINISTRATION SCHEME AND PHARMACEUTICAL USE OF AST-3424 FOR TREATING CANCERS AND TUMORS**

(30) Priority: 05.09.2023 CN 202311143390
(71) Applicant: Ascentawits Pharmaceuticals, Ltd., Shenzhen, Guangdong 518122 (CN)
(72) Inventor: DUAN, Jianxin, Shenzhen, Guangdong 518000 (CN); HAO, Jing, Shenzhen, Guangdong 518000 (CN); MENG, Fanying, San Francisco California 94121 (US); QI, Tianyang, Shenzhen, Guangdong 518000 (CN); SHAN, Qiuyue, Shenzhen, Guangdong 518000 (CN); ZHU, Qian, Shenzhen, Guangdong 518000 (CN); WANG, Ning, Shenzhen, Guangdong 518000 (CN); LIU, Nan, Shenzhen, Guangdong 518000 (CN); LI, Ye, Shenzhen, Guangdong 518000 (CN); LI, Bing, Shenzhen, Guangdong 518000 (CN)
(74) Representative: Haseltine Lake Kempner LLP
(86) International application number: PCT/CN2024/117144
(87) International publication number: WO 2025/051195

(57) **Abstract**

An administration scheme and a pharmaceutical use of AST-3424 for treating cancers and tumors, relating to a treatment method for malignant tumors, and in particular to an administration scheme and a pharmaceutical use of an AKRl C3-activated anti-cancer prodrug compound AST-3424 for treating malignant tumors, relating to the field of tumor treatment. Provided is an administration scheme of AST-3424 in monotherapy of human malignant tumors, characterized in that the interval time of two administrations within one treatment cycle is 8-144 h, preferably 12-72 h, and the number of administrations within one treatment cycle is not less than 3. Provided is a pharmaceutical use of AST-3424 in preparation of a drug for monotherapy of human malignant tumors, characterized in that the interval time of two administrations within one treatment cycle is 8-144 h, preferably 12-72 h, and the number of administrations within one treatment cycle is not less than 3.

## Description

### Technical Field

The present invention relates to a method for treating malignant tumors in the human body, and in particular to a dosing regimen and pharmaceutical use of an AKR1C3-activated anticancer prodrug compound AST-3424 for treating malignant tumors, belonging to the field of tumor treatment.

### Background Art

The chemical structure of the DNA alkylating agent prodrug AST-3424 (WO2016145092, WO2017087428) targeting overexpressed aldo-keto reductase 1C3 (AKR1C3), CAS No. 2097713-69-2, is as follows:

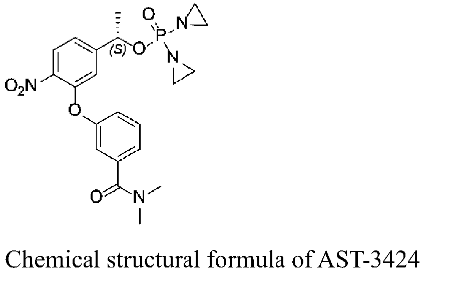

AST-3424 (also known as OBI-3424 or TH-3424) enters cancer cells and is activated by the AKR1C3 enzyme overexpressed by the cancer cell to release the metabolite AST-2660. AST-3424 itself is less toxic to cancer cells, and its pharmacological effects in animal models and *in vitro* pharmacological experiments are associated with the expression of the AKR1C3 enzyme: the prodrug AST-3424 is metabolized to AST-2660 under the action of the AKR1C3 enzyme and NADPH, and the expression level of the enzyme is positively correlated with the drug efficacy (Reference 1; Reference 2; Reference 3; Reference 4). 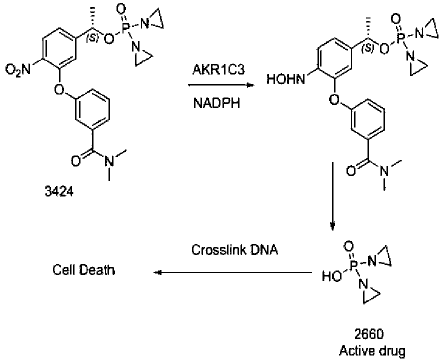 Chemical reaction formula of AST-3424 (OBI-3424) metabolized to AST-2660 (OBI-2660)

At present, the drug has entered Phase I/II clinical trials in China and the United States, respectively (NCT03592264 in the US, indication: liver cancer, pancreatic cancer and other solid tumors, sponsor: OBI Pharma Inc (4174), with the drug name OBI-3424; NCT04315324 in the US, indication: T-ALL/T-LBL (acute T-lymphoblastic leukemia/T-lymphoblastic lymphoma), with the drug name OBI-3424; CTR20191399 in China, indication: various solid tumors, sponsor: Ascentawits Pharmaceuticals, Ltd., with the drug name AST-3424; CTR20201915, indication: acute T-lymphocytic leukemia and acute B-lymphocytic leukemia, sponsor: Ascentawits Pharmaceuticals, Ltd., with the drug name AST-3424).

In these Phase I/II clinical trials conducted in China and the United States, one of the dosing regimens is a 21-day treatment cycle, with administration on Day 1 and Day 8 respectively (totaling two doses), and no administration from Day 2 to Day 7 and from Day 9 to Day 21.

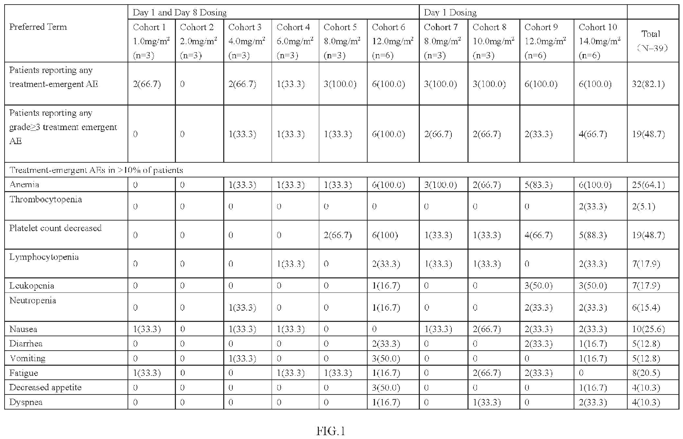

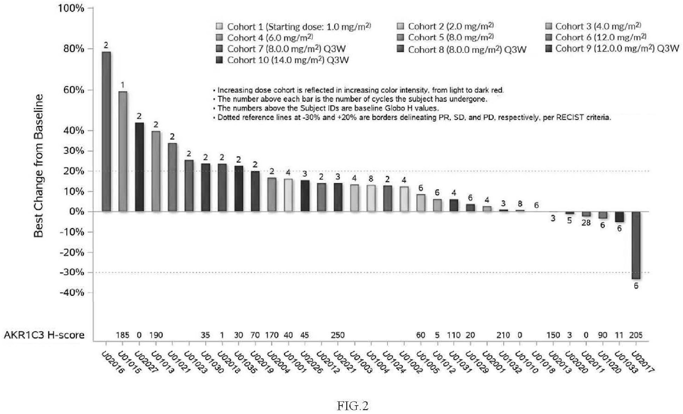

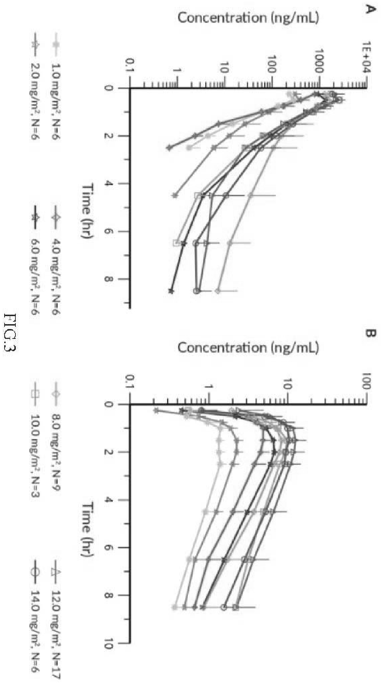

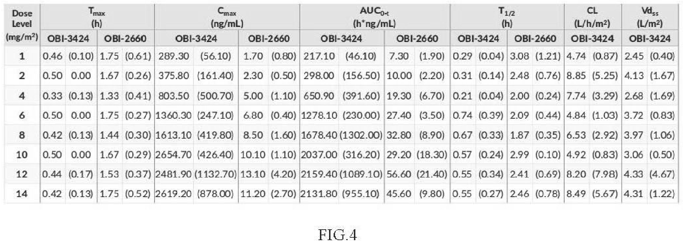

The results of the completed Phase I clinical trial in the United States are summarized as follows (specifically see the study poster in Reference 5 for details, with the Abstract section, Table 2, Figure 4, Figure 3, and Table 3 from the study poster of Reference 5 being excerpted and translated; the applicant here has translated the abstract as below and correspondingly renamed the aforementioned figures and tables into Figure 1, Figure 2, Figure 3, and Figure 4 of the present application):
Trial regimens: OBI-3424 was administered intravenously at doses of 1, 2, 4, 6, 8, or 12 mg/m² (21 days per cycle, with administration on Day 1 and Day 8, Regimen A) or at doses of 8, 10, 12, or 14 mg/m² (21 days per cycle, with administration only on Day 1, Regimen B). A "3+3" dose-escalation design was adopted. Patients continued study treatment until disease progression, development of intolerable toxicity, or completion of up to 2 years of treatment.
Results: A total of 39 adult patients were treated.

In patients receiving Regimen A, the maximum tolerated dose (MTD) of OBI-3424 was determined to be 8 mg/m²; dose-limiting toxicities (DLTs) were reported at the 12 mg/m² dose level, including thrombocytopenia (Grade 3-4 in 5 out of 6 patients) and anemia (Grade 3-4 in 5 out of 6 patients); the nadir of the platelet count was observed on Day 15 or Day 22.

In patients receiving Regimen B, the MTD was not reached at the highest tested dose (14 mg/m²); among the 6 patients treated with 14 mg/m², 3 experienced Grade 3 or higher anemia; the recommended Phase II dose (RP2D) was 12 mg/m² (21 days per cycle, with administration on Day 1).

82% (32/39) of patients experienced treatment-emergent adverse events (TRAEs). The most common TRAEs were anemia (64%), thrombocytopenia (51%), nausea (26%), and fatigue (21%). No fatal TRAEs occurred in any patients. Grade ≥3 TRAEs were reported in 49% of patients, including 3 patients who experienced serious TRAEs.

OBI-3424 showed linear pharmacokinetic characteristics at doses ranging from 1 to 14 mg/m², with minimal accumulation after repeated administration.

Evaluation via validated retrospective immunohistochemistry (IHC) indicated that 27% of patients had a high AKR1C3 staining rate (H-score ≥135). The confirmed best response in 21 patients (54%) was stable disease.

Conclusion: The researchers completed the dose-escalation portion of the clinical study of OBI-3424. The RP2D was determined to be 12 mg/m² administered once every 3 weeks (a total dose of 12 mg/m² per cycle). OBI-3424 was well-tolerated. Dose-dependent, non-cumulative thrombocytopenia and anemia were identified as dose-limiting toxicities.

Similar to AST-3424, Yangli Pharmaceutical (Vybio) and WuXi AppTec/Medshine Discovery have developed TFX05-01 (Patent Applications PCT/CN2020/120281, with Publication No. WO2021068952A1, corresponding to Chinese Application No. 202080071652.8, with Publication No. CN114555574A; and PCT/CN2021/118597, with Publication No. WO2022057838A1, corresponding to Chinese Application No. 202180064048.7, with Publication No. CN116249698A). As reported (see specifically Reference 7), it is also an AKR1C3-activated DNA alkylating agent prodrug, which is metabolized to generate DNA alkylating agent TFX05-01A (also known as AST-2660) under the combined action of NADPH and AKR1C3, as presented in a poster at the AACR 2022 Annual Meeting. Referring to the Phase I clinical trial protocols for AST-3424 and OBI-3424, this candidate drug is currently undergoing a Phase I clinical (Clinical Trial Registration No. CTR20220957) trial for injectable TFX05-01 in China: a clinical study to evaluate the safety, tolerability, pharmacokinetics, efficacy, and correlation with AKR1C3 enzyme expression of TFX05-01 in the treatment of subjects with advanced solid tumors, the dosing regimen of which is a 3-week cycle with administration on Day 1 and Day 8 of each cycle via intravenous infusion. The clinical trial plans to enroll 56 subjects, and as of now, 10 subjects have been enrolled.

### Summary of the Invention

As the sponsor of the Phase I clinical trial of AST-3424 in China, the applicant has now completed the Phase I solid tumor clinical trial conducted in China (CTR20191399) and hereby discloses and summarizes the trial results for the first time as follows:
A total of 21 subjects were enrolled in the trial. The treatment cycle was 21 days, with administration on Day 1 and Day 8. Dose escalation was conducted across five dose levels: 1.0, 2.0, 4.0, 6.0, and 8.0 mg/m². After discussion by the Safety Review Committee (SRC), the MTD and RP2D when administered on Day 1 and Day 8 of every 21-day treatment cycle were confirmed as 6.0 mg/m² (a total dose of 12 mg/m² per cycle). The safety and tolerability were slightly better in the U.S. subjects than in the Chinese subjects (with a marginally higher MTD/RP2D under the same dosing regimen than those in the Chinese population). The safety profile trends were consistent (most adverse events were mild in severity; the most common adverse events included anemia, decreased platelet count, fatigue, and vomiting). Other safety, PK and efficacy results were generally similar to those in the clinical trials of OBI-3424.

In addition to the dose exploration with a 21-day cycle and administration on Day 1 and Day 8 as described above, the clinical trials in the United States also explored a dosing regimen (doses: 8, 10, 12, or 14 mg/m²) with a 21-day cycle and administration only on Day 1. After comparison, the dosing regimen for the subsequent Phase II clinical trials conducted in the United States was ultimately determined to be a 21-day treatment cycle with administration once on Day 1 per cycle and an RP2D of 12.0 mg/m². The dosing regimen of the Phase II clinical trial determined according to the results of the Phase I clinical trials conducted in China is a 21-day treatment cycle with administration once on Day 1 and Day 8, respectively, per cycle and an RP2D of 6.0 mg/m².

As can be known from Reference 5, the Phase II clinical trial in the United States will adopt a higher dose (12 mg/m²) with administration only on Day 1 of the 21-day treatment cycle, which was derived from the comparison with administration on Day 1 and Day 8 of the 21-day treatment cycle: referring to Figure 1, at the same administration doses (8, 12 mg/m²), the probability of grade ≥3 TEAEs occurring in patients who were administered a dose of 12 mg/m² only on Day 1 was 33.3% (occurring in 2 out of 6 patients), whereas the probability of grade ≥3 TEAEs occurring in patients who were administered a dose of 12 mg/m² on Day 1 and Day 8 was 100% (occurring in all 6 patients). The applicant speculates that in order to reduce relatively severe TEAEs, the decision was made to use a high dose for RP2D and administer it once per treatment cycle.

The active ingredient of AST-3424 after activation by the AKR1C3 enzyme is AST-2660. AST-2660 is an anti-tumor compound similar to ifosfamide/DNA alkylating agents, whose efficacy is dose-dependent: higher doses can bring better efficacy but may also lead to more severe side effects and adverse reactions accordingly, as higher doses may cause drug accumulation in the body.

In order to increase the total administration dose of AST-3424 during the treatment cycle as much as possible to improve efficacy, OBI adopted a regimen of reducing the administration frequency while increasing the single administration dose. The applicant speculates that the basis for this lies in the fact that the single high-dose dosing regimen can reduce more severe TEAEs.

However, the MTD and RP2D determined in the Phase I clinical trial completed in China (every 21 days as one cycle, with administration on Day 1 and Day 8) were 6.0 mg/m², which was slightly lower than the MTD (8.0 mg/m²) administered via the same regimen in the Phase I clinical trial in the United States.

Based on the above differences, the approach adopted for enhancing therapeutic efficacy in certain patients receiving AST-3424 while minimizing the occurrence and severity of TEAEs should be high-frequency, low-dose administration:
*the higher the total exposure of the chemotherapy drugs per cycle, the better the efficacy;
*the higher the single administration dose of the chemotherapy drugs, the greater the toxicity;
*to improve efficacy and reduce toxicity, low-dose, multiple administrations should be adopted.

To determine the administration interval, the human pharmacokinetic data in China and the United States were investigated.

As shown in Figures 3 and 4, in clinical trials conducted in the United States, the drug OBI-3424 rapidly reached the peak plasma concentration after intravenous injection, with a T₁/₂ of approximately 0.4 hours; in contrast, the parent drug's metabolite AST-2660 showed a delayed time-to-peak, with a T₁/₂ of approximately 2.3 hours.

In light of the control data from the AST-3424 clinical trial conducted in China, the metabolic profiles of AST-3424 (OBI-3424) and AST-2660 (OBI-2660) in Chinese and American patients are similar, with minimal differences in t1/2, wherein the concentrations of AST-3424 (OBI-3424) and AST-2660 (OBI-2660) are almost lower than 1ng/ml after 8 hours, i.e. there is almost no accumulation of drugs (including AST-3424 and AST-2660) after 8 hours.

In particular, the Cmax of AST-3424 (OBI-3424) in the bloodstream is hundreds of times that of AST-2660 (OBI-2660) in the bloodsteam, which also indirectly indicates that most of the metabolism of AST-3424 (OBI-3424) occurs intracellularly, and only a very small proportion of AST-3424 is activated and metabolized to AST-2660 (OBI-2660) in the bloodstream.

The process by which AKR1C3 activates the prodrug AST-3424 (OBI-3424) to AST-2660 (OBI-2660) in the human body is rapid in both Chinse and American patients.

Based on the blood pharmacokinetic experimental data obtained from clinical trials in Chinese and American patients, the inventors believe that when the interval between two administrations is 8 hours or more, the use of a new regimen involving consecutive multiple administrations is expected to increase the total dose administered in one treatment cycle, thereby enhancing the efficacy. Meanwhile, according to the observed temporal pattern of platelet count decline, patients will have a longer recovery period, which may achieve the effect of reducing the incidence or severity of related adverse reactions.

To this end, a new dosing regimen and pharmaceutical use are proposed below for AKR1C3-activated DNA alkylating prodrug compounds, including AST-3424 (OBI-3424)/TFX05-01, which is metabolized to AST-2660.

The dosing regimen for AKR1C3-activated DNA alkylating prodrug compounds that are metabolized to AST-2660 for the treatment of human tumors as monotherapy or in combination is characterized by an interval of 8-144 hours, preferably 12-72 hours, between two consecutive administrations within one treatment cycle, with no fewer than 3 administrations within one treatment cycle. The tumors are preferably malignant tumors.

The pharmaceutical use of AKR1C3-activated DNA alkylating prodrug compounds that are metabolized to AST-2660 for preparing a medicament for the treatment of human tumors as monotherapy or in combination is characterized by an interval of 8-144 hours, preferably 12-72 hours, between two consecutive administrations within one treatment cycle, with no fewer than 3 administrations within one treatment cycle. The tumors are preferably malignant tumors.

The prodrugs activated by the AKR1C3 enzyme are selected from compounds of formulae (1)-(6): 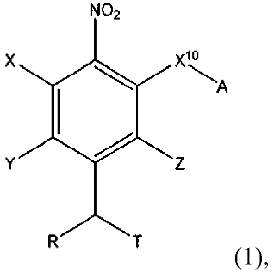 wherein X, Y, Z, R, A and X¹⁰ are as defined in the claims of Patent Application PCT/US2016/021581, with Publication No. WO2016145092A1 (corresponding to Chinese Application No. 2016800150788, with Publication No. CN107530556A); T is 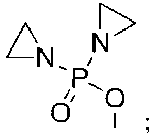 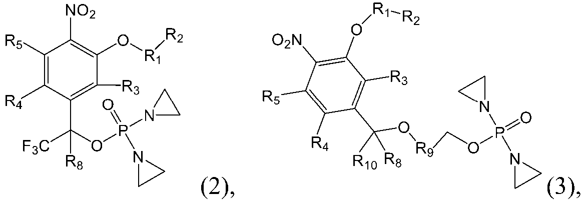 wherein R₁, R₂, R₃, R₄, R₅, R₈, R₉ and R₁₀ are as defined in the claims of Patent Application PCT/CN2020/089692, with Publication No. WO2020228685A9 (corresponding to Chinese Application No. 2020800358890, with Publication No. CN113853379A); 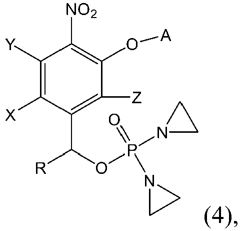 wherein:
A is substituted or unsubstituted C₆-C₁₀ aryl, biaryl, or substituted biaryl, 5-15 membered heteroaryl, or -N=CR¹R², wherein a substituent for substitution is selected from the group consisting of: halogen, -CN, -NO₂, -O-(CH₂)-O-, -CO₂H and salts thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, - SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5-7 membered heterocyclic ring;
wherein the alkyl and aryl are each substituted by 1-3 halogens or 1-3 C₁-C₆ alkyls;
R¹ and R² are each independently phenyl or methyl;
X, Y and Z are each independently hydrogen or halogen;
R is hydrogen or C₁-C₆ alkyl or halogen-substituted alkyl; 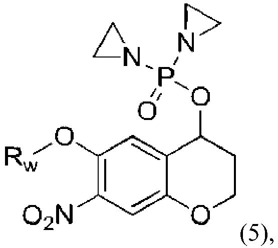
wherein Rw is as defined in the claims of Patent Application PCT/CN2020/120281, with Publication No. WO2021068952A1 (corresponding to Chinese Application No. 202080071652.8, with Publication No. CN114555574A); 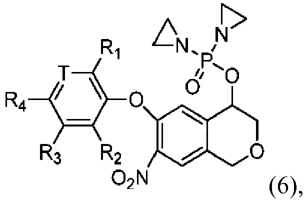
wherein R₁, R₂, R₃, R₄ and T are as defined in the claims of Patent Application PCT/CN2021/118597, with Publication No. WO2022057838A1.

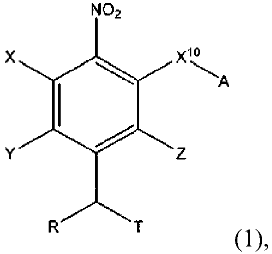

Formula (1) is an AKR1C3 enzyme-activated alkylating agent prodrug compound, which, more specifically, is an AKR1C3 enzyme-activated DNA alkylating agent prodrug compound;
wherein X, Y, Z, R, T, A, and X¹⁰ are as defined in the claims of Patent Application PCT/US2016/021581, with Publication No. WO2016145092A1 (corresponding to the Chinese Application No. 2016800150788, with Publication No. CN107530556A), and the synthetic preparation methods for specific compounds are also described in the above application, which is incorporated herein by reference in its entirety; the detailed definitions are as follows:
X¹⁰ is O, S, SO, or SO₂;
A is C₆-C₁₀ aryl, 5- to 15-membered heteroaryl or-N=CR¹R²;
R¹ and R² are each independently hydrogen, C₁-C₆ alkyl, C₃-C₈ cycloalkyl, C₆-C₁₀ aryl, 4- to 15-membered heterocycle, ether, -CONR¹³R¹⁴ or -NR¹³COR¹⁴;
X, Y and Z are each independently hydrogen, CN, halogen, C₁-C₆ alkyl, C₂-C₆ alkenyl, C₂-C₆ alkynyl, C₃-C₈ cycloalkyl, C₆-C₁₀ aryl, 4- to 15-membered heterocycle, ether, - CONR¹³R¹⁴ or -NR¹³COR¹⁴;
R is hydrogen, C₁-C₆ alkyl, C₂-C₆ alkenyl, C₂-C₆ alkynyl, C₃-C₈ cycloalkyl, C₆-C₁₀ aryl, 4- to 15-membered heterocycle, ether, -CONR¹³R¹⁴ or -NR¹³COR¹⁴;
R¹³ and R¹⁴ are each independently hydrogen, C₁-C₆ alkyl, C₃-C₈ cycloalkyl, C₆-C₁₀ aryl, 4- to 15-membered heterocyclic ring or ether;
T is 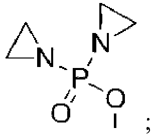 and
wherein these alkyl, alkenyl, alkynyl, cycloalkyl, aryl, heterocyclyl, heteroaryl, and ether are substituted or unsubstituted. 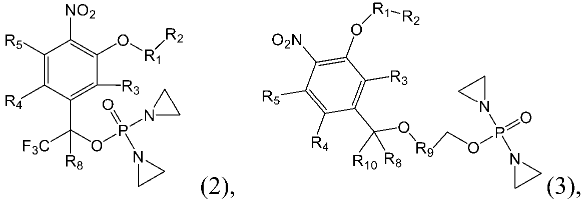
wherein R₁, R₂, R₃, R₄, R₅, R₈, R₉, and R₁₀ are as defined in the claims of Patent Application PCT/CN2020/089692, with Publication No. WO2020228685A1, and the synthetic preparation methods of specific compounds are also described in the above application, which is incorporated herein by reference in its entirety; the detailed definitions are as follows:
   R₁ is C₆-C₁₀ aryl or Z-substituted aryl, a 4- to 15-membered heterocycle or Z-substituted heterocycle, a 5- to 15-membered heteroaryl or Z-substituted heteroaryl, or a 7- to 15-membered fused ring or Z-substituted fused ring;
   R₂ is hydrogen, a halogen atom, cyano or isocyano, hydroxy, sulfhydryl, amino, OTs, OMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, a 4- to 15-membered heterocycle or Z-substituted heterocycle, a 5- to 15-membered heteroaryl or Z-substituted heteroaryl, an ether having 1 to 6 carbon atoms or a Z-substituted alkoxy having from 1 to 6 carbon atoms, -CONR⁶R⁷, - SO₂NR⁶R⁷, -SO₂R⁶, -OCOO-R⁶, -COOR⁶, -NR⁶COR⁷, -OCOR⁶, -NR⁶SO₂R⁷ or - NR⁶SO₂NR⁶R⁷, or R² together with the atom in the group R¹ to which it is bonded forms a 7- to 15-membered fused ring or Z-substituted fused ring;
   R₃ is hydrogen, halogen, cyano or isocyano, hydroxy, sulfhydryl, amino, OTs, OMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, a 4- to 15-membered heterocycle or Z-substituted heterocycle, a 5- to 15-membered heteroaryl or Z-substituted heteroaryl, C₁-C₆ alkoxy or Z-substituted C₁-C₆ alkoxy, -CONR⁶R⁷, -SO₂NR⁶R⁷, -SO₂R⁶, -OCO-R⁶, -OCOO-R⁶, - COOR⁶, -NR⁶COR⁷, -OCOR⁶, or -NR⁶SO₂R⁷;
   R₄ and R₅ are each independently hydrogen, a halogen atom, cyano or isocyano, hydroxy, sulfhydryl, amino, OTs, OLCMS, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, a 4- to 15-membered heterocycle or Z-substituted heterocycle, a 5- to 15-membered heteroaryl or Z-substituted heteroaryl, C₁-C₆ alkoxy or Z-substituted C₁-C₆ alkoxy, -CONR⁶R⁷, - SO₂NR⁶R⁷, -SO₂R⁶, -OCOO-R⁶, -COOR⁶, -NR⁶COR⁶, -OCOR⁶ or -NR⁶SO₂R⁷, or R⁴ and R⁵ together with the atom of the benzene ring to which they are bonded to form a 7- to 15-membered fused ring or Z-substituted fused ring;
   R₆ and R₇ are each independently hydrogen, cyano or isocyano, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, C₆-C₁₀ aryl or Z-substituted aryl, a 4- to 15-membered heterocycle or Z-substituted heterocycle, a 5- to 15-membered heteroaryl or Z-substituted heteroaryl, C₁-C₆ alkoxy or Z-substituted C₁-C₆ alkoxy, or R⁶ and R⁷ together with the atom to which they are bonded to form a 5- to 7-membered heterocyclyl or Z-substituted 5- to 7-membered heterocyclyl;
   R₈ and R₁₀ are each independently hydrogen, deuterium, aryl or Z-substituted aryl, C₁-C₆ alkyl or Z-substituted alkyl, C₂-C₆ alkenyl or Z-substituted alkenyl, C₂-C₆ alkynyl or Z-substituted alkynyl, C₃-C₈ cycloalkyl or Z-substituted cycloalkyl, with the proviso that at least one of R⁸ and R¹⁰ must be hydrogen or deuterium;
   R₉ is substituted C₆-C₁₀ aryl which is substituted with at least one fluorine atom or nitro group, substituted 4- to 15-membered heterocycle which is substituted with at least one fluorine atom or nitro group, or substituted 5- to 15-membered heteroaryl which is substituted with at least one fluorine atom or nitro group;
   the substituent Z is a halogen atom, cyano or isocyano, hydroxy, sulfhydryl, amino, OTs, OMS, C₁-C₃ alkyl or substituted alkyl, C₁-C₃ alkoxy or substituted alkoxy, C₂-C₃ alkenyl or substituted alkenyl, C₂-C₃ alkynyl or substituted alkynyl, C₃-C₈ cycloalkyl or substituted cycloalkyl, an aromatic ring, a heterocycle, a heteroaromatic ring and a fused ring or a substituted aromatic ring, heterocycle, heteroaromatic ring and fused ring, with the substitution pattern being mono- or gem-di-substitution;
   in R₉, the substituent on the substituted C₆-C₁₀ aryl, substituted 4- to 15-membered heterocycle or substituted 5- to 15-membered heteroaryl is a halogen atom, nitro, cyano or isocyano, hydroxy, amino, C₁-C₃ alkyl or alkoxy, alkenyl, alkynyl, cycloalkyl or a benzene ring, a substituted benzene ring, or C₁-C₃ alkoxy or a halogen atom-substituted alkoxy.

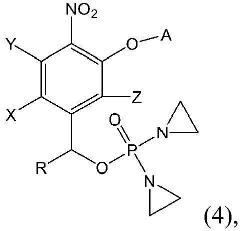

Formula (4) is an AKR1C3 enzyme-activated alkylating agent prodrug compound, which, more specifically, is an AKR1C3 enzyme-activated DNA alkylating agent prodrug compound;
wherein:
A is substituted or unsubstituted C₆-C₁₀ aryl, biaryl, or substituted biaryl, a 5- to 15-membered heteroaryl, or -N=CR¹R², wherein the substituent is selected from the group consisting of: halogen, -CN, -NO₂, -O-(CH₂)-O-, -CO₂H and salts thereof, -OR¹⁰⁰, - CO₂R¹⁰⁰, -CONR¹⁰¹R¹⁰², -NR¹⁰¹R¹⁰², -NR¹⁰⁰SO₂R¹⁰⁰, -SO₂R¹⁰⁰, -SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5- to 7-membered heterocyclic ring;
wherein alkyl and aryl are each substituted by 1-3 halogens or 1-3 C₁-C₆ alkyls;
R¹ and R² are each independently phenyl or methyl;
X, Y and Z are each independently hydrogen or halogen;
R is hydrogen C₁-C₆ alkyl, or a halogen-substituted alkyl. 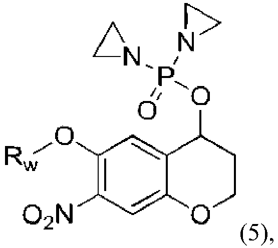
wherein Rw is as defined in the claims of Patent Application PCT/CN2020/120281, with Publication No. WO2021068952A1, and the synthetic preparation methods of specific compounds are also described in the above application, which is incorporated herein by reference in its entirety; the detailed definitions are as follows:
   Rw is 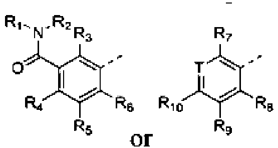
   R₁ is H, C₁₋₆ alkyl, C₃₋₆ cycloalkyl, 4- to 6-membered heterocycloalkyl, 5- to 6-membered heteroaryl or phenyl, wherein the C₁₋₆ alkyl, C₃₋₆ cycloalkyl, 4- to 6-membered heterocycloalkyl, 5- to 6-membered heteroaryl and phenyl are optionally substituted with 1, 2 or 3 R^{a} groups;
   each R^{a} is independently H, F, Cl, Br, I, -CN, -OH, C₁₋₃ alkoxy or C₁₋₃ alkyl;
   R₂ is H or C₁₋₆ alkyl;
   or R₁ and R₂, together with the N atom to which they are attached, form a 4- to 6-membered heterocycloalkyl, wherein the 4- to 6-membered heterocycloalkyl is optionally substituted with 1, 2 or 3 R^{b} groups;
   each R^{b} is independently H, F, Cl, Br, I, -CN, -OH, -NH₂, -OCH₃, -OCH₂CH₃, -CH₃ or -CH₂CH₃;
   R₃ is H, F, Cl, Br, I, -OH, -NH₂, C₁₋₃ alkoxy or C₁₋₃ alkyl;
   or R₂ and R₃ are attached together such that the structural unit 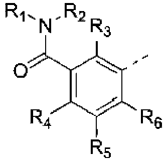 is 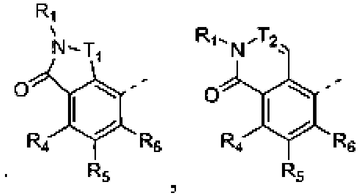 or 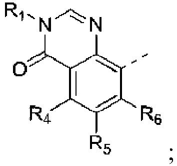
   T₁ is -(CR^{c}R^{d})ₘ- or -(CR^{c}R^{d})ₙ-O-;
   m is 1, 2 or 3;
   n is 1 or 2;
   T₂ is N or CH;
   R^{c} and R^{d} are each independently H, F, C₁₋₃ alkyl or C₁₋₃ alkoxy;
   R₄, R₅ and R₆ are each independently H, F, Cl, Br, I, C₁₋₃ alkyl or C₁₋₃ alkoxy;
   T is N or CH;
   R₇ and R₈ are each independently H, F, Cl, Br or I;
   R₉ and R₁₀ are each independently H, F, Cl, Br, I, -CN; or
   the 4- to 6-membered heterocycloalkyl and 5- to 6-membered heteroaryl each contain 1, 2, 3 or 4 heteroatoms independently selected from N, -O- and -S-. 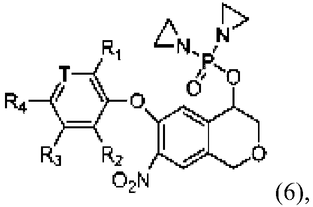
   wherein R₁, R₂, R₃, R₄ and T are as defined in the claims of Patent Application PCT/CN2021/118597, with Publication No. WO2022057838A1, and the synthetic preparation methods of specific compounds are also described in the above application, which are incorporated herein by reference in their entirety; the detailed definitions are as follows:
      T is N or CH;
      R₁ and R₂ are each independently H, F, Cl, Br, I or C₁₋₃ alkyl, wherein said C₁₋₃ alkyl is optionally substituted with 1, 2 or 3 Rₐ;
      each Rₐ is independently F, Cl, Br, I, -CN, -OH, or -NH₂;
      R₃ and R₄ are each independently H, F, Cl, Br, I, CN, C₁₋₃ alkyl, C₁₋₃ alkoxy, 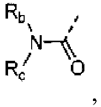 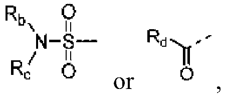 wherein the C₁₋₃ alkyl is optionally substituted with 1, 2 or 3 Rₑ;
      R_{b} and R_{c} are each independently H, -CH₃, -CH₂CH₃, -(CH₂)₂CH₃, or -CH (CH₃)₂;
      R_{d} is -CH₃, -CH₂CH₃, -(CH₂)₂CH₃, or -CH(CH₃)₂;
      each Rₑ is independently F, Cl, Br, I, -CN, -OH, or -NH₂.

In particular, these AKR1C3-activated DNA alkylating agent prodrug compounds that are metabolized to AST-2660 are selected from AST-3424 and TFX05-01.

TFX05-01 is selected from one of the following structural compounds:
Compounds of structural formula (5) 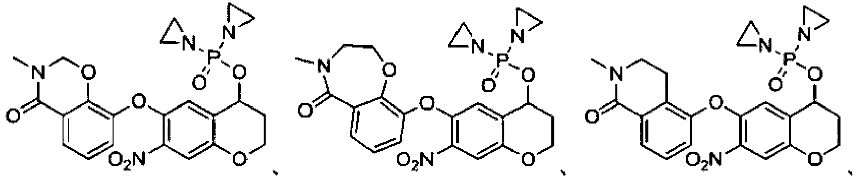 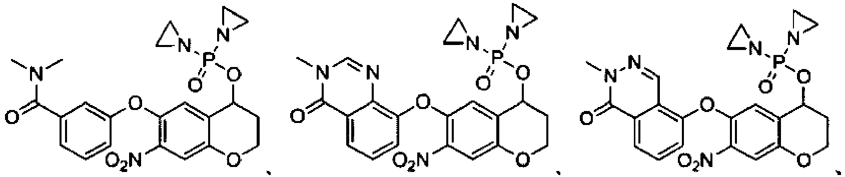 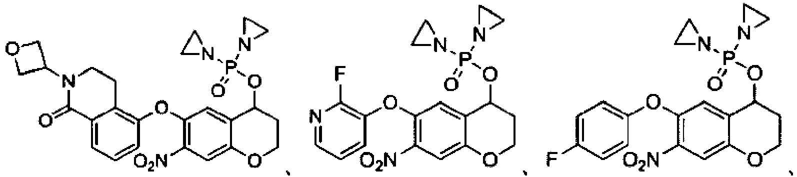 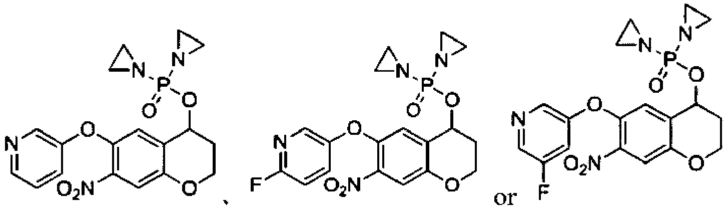 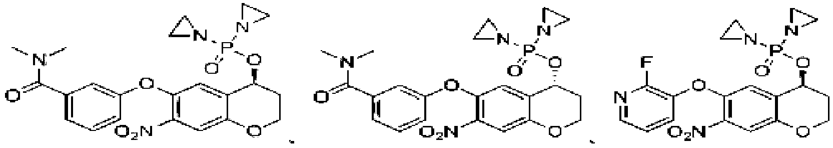 or 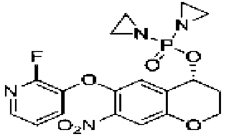
Compounds of structural formula (6)

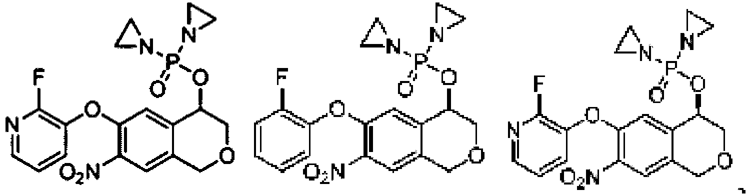

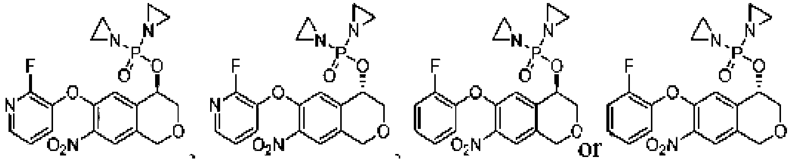

The dosing regimen of AST-3424 as monotherapy or combination therapy for human malignant tumors is characterized in that an interval between two consecutive administrations within one treatment cycle is 8-144 hours.

Preferably, the administration interval is 12-72 hours. More preferably, the number of administrations within one treatment cycle is not less than 3.

The pharmaceutical use of AST-3424 in the manufacture of a medicament for the treatment of human tumors as monotherapy or combination therapy is characterized in that an interval between two consecutive administrations within one treatment cycle is 8-144 hours.

Preferably, the administration interval is 12-72 hours. More preferably, the number of administrations within one treatment cycle is no less than 3.

The tumors are preferably malignant tumors.

Based on the cellular characteristics of neoplasms and the degree of harm they cause to the body, tumors are further classified into two major categories: benign tumors and malignant tumors. Malignant tumors can be divided into carcinoma and sarcoma. Carcinoma refers to malignant tumors originating from epithelial tissues. Sarcoma refers to malignant tumors arising from mesenchymal tissues, including fibrous connective tissue, adipose tissue, muscle, vasculature, bone, and cartilage tissues.

AST-3424 refers to a pharmaceutical preparation comprising AST-3424, i.e., a pharmaceutical formulation or pharmaceutical product (distinguished from the active pharmaceutical ingredient, API) that can be directly administered to human patients.

The term "pharmaceutical" as described herein refers to a pharmaceutical product or preparation. The prepared pharmaceutical product contains the active ingredient AST-3424 within a specific dosage range, and the prepared medicament is administered in a specific dosage form and in a specific mode of administration.

The prepared pharmaceutical product, medicament, and preparation may also contain pharmaceutically acceptable adjuvants or excipients. The drug may be in any dosage form for clinical administration, such as tablets, suppositories, dispersible tablets, enteric-coated tablets, chewable tablets, orally disintegrating tablets, capsules, sugarcoated tablets, granules, dry powders, oral solutions, ampoules for injection, lyophilized powder injections, or large infusions. Depending on the specific dosage form and mode of administration, the pharmaceutically acceptable adjuvants or excipients in the drug may include one or more of the following: diluents, solubilizers, disintegrants, suspending agents, lubricants, binders, fillers, flavoring agents, sweetening agents, antioxidants, surfactants, preservatives, encapsulating agents, and pigments.

The currently available dosage form is an injectable preparation, specifically the AST-3424 concentrated solution for injection disclosed in Patent Application PCT/CN2020/101870, with Publication No. WO2021008520 (corresponding to Chinese Application CN202080001484.5, with Publication No. CN112469394A). This solution is diluted before use and administered by intravenous injection, preferably by intravenous infusion. Naturally, other dosage forms may be developed subsequently.

Monotherapy refers to single drug therapy. Monotherapy herein further requires that the drug contains only the active ingredient AST-3424 and does not contain other active ingredients; that is, the drug is not a compound drug. Combination therapy refers to combined drug therapy. Single drug therapy refers to the use of only one anticancer drug in a course of treatment. Combination therapy refers to the simultaneous or sequential use of two or more anticancer drugs in a course of treatment.

Drugs used in combination with AST-3424 may be other anticancer drugs. The literature has proven that the use of AST-3424 in combination with abiraterone, prednisolone, 5-fluorouracil, sunitinib (WO2022178821), immune checkpoint inhibitors such as PD-1/L1 (WO2022231580), nelarabine (WO2019062919), or oxaliplatin/5-fluorouracil (Reference 6) exhibits a relatively significant combination therapy effect.

The interval between two consecutive administrations within one treatment cycle is 8-144 hours. The minimum value of 8 hours within the range of 8-144 hours is derived based on the blood drug metabolism data from the aforementioned Phase I clinical trials of AST-3424 (OBI-3424) conducted in China and the United States. However, it is evident that the administration interval cannot be indefinitely extended, and the inventors recommend a maximum interval of 6 days, i.e., 144 hours.

Since AST-2660, which is a DNA alkylating agent, can be observed in the blood and may cause toxic side effects on the blood or other human tissues, efforts should be made to prevent AST-2660 from accumulating in the blood to a high concentration. Moreover, according to the aforementioned drug metabolism results, AST-2660 basically disappears from the blood after 8 hours, so the minimum administration interval is set at 8 hours.

Chemotherapy is generally calculated in cycles, typically 21 days or 28 days per cycle, and of course, there are also cycles of 14 days or even 7 days: that is, the interval from the start of administration on Day 1 to the start of the next cycle of chemotherapy is 21 days, 28 days, 14 days or even 7 days. Because chemotherapy can not only kill tumor cells but also some normal cells, the body needs a certain amount of time to recover. Meanwhile, chemotherapy can also cause myelosuppression, and blood indicators also need to recover to a certain level before the next cycle of treatment can be carried out. Therefore, one treatment cycle of chemotherapy can be divided into an administration period and a recovery period: during the administration period, a drug is administered either continuously or intermittently, and a certain interval is required between two administrations, which is related to the nature of the drug itself; no drug is administered during the recovery period. Here, continuous administration refers to continuous daily administration during the administration period, and intermittent administration refers to administration with an interval of at least 1 day during the administration period.

The duration of one treatment cycle is determined through comparison of clinical trials. For most chemotherapy drugs, a 21- or 28-day cycle not only allows the body to recover but also prevents excessively long intervals that could allow tumor cells to re-proliferate or grow. Hence, chemotherapy cycles are typically 21 or 28 days.

Within one treatment cycle, the period from the first administration to the last administration is defined as the administration period in the present application, while the remaining time in the remaining treatment cycle is the recovery period. Generally speaking, the recovery period of chemotherapy is greater than or equal to the administration period, which ensures sufficient time for the patient's body to recover naturally.

AST-3424 (OBI-3424) is a prodrug of the chemotherapy drug AST-2660, and it is the chemotherapy drug AST-2660 that exerts the therapeutic effect in vivo. Since AST-2660 is a chemotherapy drug similar to ifosfamide, its therapeutic administration also conforms to the cyclical patterns of chemotherapy drugs.

As a preference, the interval between two consecutive administrations within one treatment cycle is 8, 12, 24, 48, 72, 96, 120, or 144 hours. The interval here is determined by considering the blood metabolism data from the aforementioned Phase I clinical trial and in conjunction with the specific characteristics of the drug: this interval neither results in excessive accumulation of the drug in the blood nor causes the concentration of the drug in the blood to become too low to form a concentration gradient between the inside and outside of cells, thereby avoiding the failure of the drug to enter cancer cells to be activated by the AKR1C3 enzyme to exert its efficacy.

An 8-hour interval corresponds to administering the drug 3 times a day; a 12-hour interval corresponds to administering the drug twice a day; a 24-hour interval corresponds to administering the drug once a day, and so on. This is a case of uniform interval administration.

Actually, considering that patients have different physical conditions or other situations may occur, the actual administration interval is not necessarily so uniform and strict. There may be irregular administration intervals such as administration on Days 1, 2, 4, and 5. Therefore, the interval between two consecutive administrations (8, 12, 24, 48, 72, 96, 120, and 144 hours) includes not only the above-mentioned uniform interval administration, but also irregular interval dosing regimens. The same applies to the interval of 8-144 hours between two consecutive administrations within one treatment cycle.

The tumors include solid tumors and hematological tumors. The solid tumors are selected from liver cancer, colorectal cancer, gastric cancer, intrahepatic cholangiocarcinoma, pancreatic cancer, submandibular gland carcinoma, prostate cancer, breast cancer, and melanoma; the hematological tumors are selected from acute lymphoblastic leukemia, preferably acute T-lymphoblastic leukemia and acute B-lymphoblastic leukemia (T-ALL, B-ALL). All the tumor types listed above have enrolled patients in Phase I clinical trials in China and the United States.

More preferably, each treatment cycle comprises an administration period and a recovery period. During the administration period, the drug is administered continuously or intermittently with intervals of 12, 24, 48, or 72 hours between consecutive administrations. The remaining time is the recovery period, during which no drug is administered. The administration route is intravenous drip, with a dose of 1-8 mg/m², preferably 1-6 mg/m² per administration. In clinical trials, AST-3424 (OBI-3424) is administered via intravenous infusion over 30 minutes (±5 minutes). Specifically, the concentrated injection solution disclosed in Patent Application PCT/CN2020/101870, with Publication No. WO2021008520 (corresponding to Chinese Application CN202080001484.5, with Publication No. CN112469394A) is used. For use, the pH of a 5% glucose injection is adjusted to 7.4 with a sodium bicarbonate solution to prepare an isotonic solution.

A single administration is typically completed via intravenous infusion within 20-60 minutes.

The dose of 1-8 mg/m² corresponds to the single dose for the general population, and the body surface area can be calculated based on relevant body surface area formulas. The body surface area of Chinese individuals generally ranges from 1.5 to 2.0 square meters, while it may be approximately 1.3-1.4 square meters for some lean elderly women. The single dose can then be obtained based on the body surface area calculated.

When one treatment cycle is 21 days, the specific regimens are selected from the following:
administering once daily on Days 1, 2, and 3 (totaling 3 administrations) respectively, with the remaining 18 days as a recovery period without administration;
administering once daily on Days 1, 2, 3, and 4 (totaling 4 administrations) respectively, with the remaining 17 days as a recovery period without administration;
administering once daily on Days 1, 2, 3, 4, and 5 (totaling 5 administrations) respectively, with the remaining 16 days as a recovery period without administration;
administering once daily on Days 1, 2, 3, 4, 5, and 6 (totaling 6 administrations) respectively, with the remaining 15 days as a recovery period without administration;
administering once daily on Days 1, 2, 3, 4, 5, 6, and 7 (totaling 7 administrations) respectively, with the remaining 14 days as a recovery period without administration;
administering once daily on Days 1, 3, and 5 (totaling 3 administrations) respectively, with the remaining 18 days (i.e. the drug-free Day 2, Day 4, and Days 6-21, totaling 18 days; the same calculation method applies below) as a recovery period without administration;
administering once daily on Days 1, 3, 5, and 7 (totaling 4 administrations) respectively, with the remaining 17 days as a recovery period without administration;
administering once daily on Days 1, 3, 5, 7, and 9 (totaling 5 administrations) respectively, with the remaining 16 days as a recovery period without administration;
administering once daily on Days 1, 4, and 7 (totaling 3 administrations) respectively, with the remaining 18 days as a recovery period without administration;
administering once daily on Days 1, 5, and 9 (totaling 3 administrations) respectively, with the remaining 18 days as a recovery period without administration.

When one treatment cycle is 14 days, the specific regimens are selected from the following:
administering once daily on Days 1, 2, and 3 (totaling 3 administrations) respectively, with the remaining 11 days as a recovery period without administration;
administering once daily on Days 1, 2, 3, and 4 (totaling 4 administrations) respectively, with the remaining 10 days as a recovery period without administration;
administering once daily on Days 1, 2, 3, 4, and 5 (totaling 5 administrations) respectively, with the remaining 9 days as a recovery period without administration;
administering once daily on Days 1, 2, 3, 4, 5, and 6 (totaling 6 administrations) respectively, with the remaining 8 days as a recovery period without administration;
administering once daily on Days 1, 2, 3, 4, 5, 6 and 7 (totaling 7 administrations) respectively, with the remaining 7 days as a recovery period without administration;
administering once daily on Days 1, 3, and 5 (totaling 3 administrations) respectively, with the remaining 11 days as a recovery period without administration;
administering once daily on Days 1, 3, 5, and 7 (totaling 4 administrations) respectively, with the remaining 10 days as a recovery period without administration;
administering once daily on Days 1, 3, 5, 7, and 9 (totaling 5 administrations) respectively, with the remaining 9 days as a recovery period without administration;
administering once daily on Days 1, 4, and 7 (totaling 3 administrations) respectively, with the remaining 11 days as a recovery period without administration.

When one treatment cycle is 28 days, the specific regimens are selected from the following:
administering once daily on Days 1, 2, and 3 (totaling 3 administrations) respectively, with the remaining 25 days as a recovery period without administration;
administering once daily on Days 1, 2, 3, and 4 (totaling 4 administrations) respectively, with the remaining 24 days as a recovery period without administration;
administering once daily on Days 1, 2, 3, 4, and 5 (totaling 5 administrations) respectively, with the remaining 23 days as a recovery period without administration;
administering once daily on Days 1, 2, 3, 4, 5, and 6 (totaling 6 administrations) respectively, with the remaining 22 days as a recovery period without administration;
administering once daily on Days 1, 2, 3, 4, 5, 6, and 7 (totaling 7 administrations) respectively, with the remaining 21 days as a recovery period without administration;
administering once daily on Days 1, 2, 3, 4, 5, 6, 7 and 8 (totaling 8 administrations) respectively, with the remaining 20 days as a recovery period without administration;
administering once daily on Days 1, 2, 3, 4, 5, 6, 7, 8 and 9 (totaling 9 administrations) respectively, with the remaining 19 days as a recovery period without administration;
administering once daily on Days 1, 2, 3, 4, 5, 6, 7, 8, 9 and 10 (totaling 10 administrations) respectively, with the remaining 18 days as a recovery period without administration;
administering once daily on Days 1, 3, and 5 (totaling 3 administrations) respectively, with the remaining 25 days as a recovery period without administration;
administering once daily on Days 1, 3, 5, and 7 (totaling 4 administrations) respectively, with the remaining 24 days as a recovery period without administration;
administering once daily on Days 1, 3, 5, 7, and 9 (totaling 5 administrations) respectively, with the remaining 23 days as a recovery period without administration;
administering once daily on Days 1, 4, and 7 (totaling 3 administrations) respectively, with the remaining 25 days as a recovery period without administration;
administering once daily on Days 1, 4, 7 and 10 (totaling 4 administrations) respectively, with the remaining 24 days as a recovery period without administration;
administering once daily on Days 1, 4, 7, 10 and 13 (totaling 5 administrations) respectively, with the remaining 23 days as a recovery period without administration;
administering once daily on Days 1, 5, and 9 (totaling 3 administrations) respectively, with the remaining 25 days as a recovery period without administration;
administering once daily on Days 1, 5, 9 and 13 (totaling 4 administrations) respectively, with the remaining 24 days as a recovery period without administration.

Among the aforementioned regimens, for the regiments involving consecutive daily administration such as on Days 1, 2, and 3, the interval between two consecutive administrations is 24 hours; for regimens involving administration every other day such as on Days 1, 3, and 5, the interval between two consecutive administrations is 48 hours; for the regimens involving administration with a two-day interval, such as on Days 1, 4, and 7, the interval between two consecutive administrations is 72 hours; and for the regimens involving administration with a three-day interval, such as on Days 1, 5, and 9, the interval between two consecutive administrations is 96 hours. However, due to various reasons, the actual administration may not strictly adhere to the intervals of 24, 48, 72, or 96 hours and may be advanced or delayed.

Based on the Phase I clinical trials conducted in China and the United States, the dose per administration in the aforementioned regimens is 1-8 mg/m². The specific dose for monotherapy will be determined based on specific clinical trials, and is related to the specific tumor type and the like. The specific dose for combination therapy will be determined based on specific clinical trials, and is related to factors such as the specific tumor type, the types of drugs used in combination and the sequence of drug administration during combination therapy.

In particular, unless otherwise specified, all numerical values in the description have a tolerance range of ±10%. That is to say, if a certain numerical range in the description is 2-3, in practice, due to the existence of measurement errors, accidental errors, and systematic errors, if a value of 1.8-3.3 is obtained through actual measurement or calculation, it shall also be deemed to fall within the aforementioned range of 2-3 and meet the requirements.

Both the Phase I clinical trials of AST-3424 (OBI-3424) conducted in China and the United States disclosed in the above summary of the invention have been approved by the drug regulatory authorities in their respective locations, and the relevant clinical trials have been completed by clinical research institutions. The protocols of the relevant clinical trials have all been reviewed and approved by ethical committees, and the process of the clinical trials complies with the relevant medical ethics requirements, laws and regulations in the locations where the clinical trials are conducted.

### Brief Description of the Drawings

Figure 1 shows the statistics of TEAEs in patients enrolled in the Phase I solid tumor clinical trial of OBI-3424 (excerpted from Table 2 in the study poster of Reference 5 and translated into Chinese).
Figure 2 shows the preliminary treatment efficacy statistics in patients enrolled in the Phase I solid tumor clinical trial of OBI-3424 (excerpted from figure 4 in the study poster of Reference 5 and translated into Chinese; this figure is also figure 5 in the study article of Reference 5).
Figure 3 shows the pharmacokinetic profiles of OBI-3424 (A) and OBI-2660 (B) during Cycle 1 in patients enrolled in the Phase I solid tumor clinical trial of OBI-3424 (data from Day 1 and Day 8 combined) (excerpted from figure 3 in the study poster of Reference 5 and translated into Chinese; this figure is also figure 4 in the study article of Reference 5).
Figure 4 shows the pharmacokinetic parameters of OBI-3424 and OBI-2660 after intravenous infusion of OBI-3424 in patients enrolled in the Phase I solid tumor clinical trial of OBI-3424 (excerpted from Table 3 in the study poster of Reference 5 and translated into Chinese), where "dose level" represents the dose level.
Figure 5 shows the tumor growth curves of the HuPrime^{®} liver cancer LI6643 subcutaneous xenograft model in BALB/c nude mice after treatment with sorafenib and AST-3424.

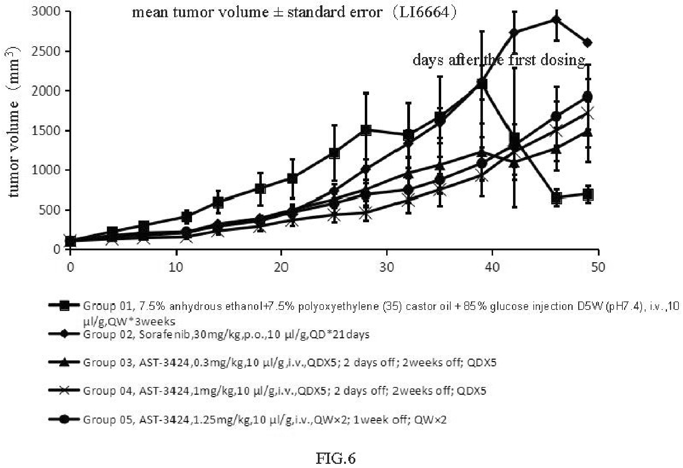

Figure 6 shows the tumor growth curves of the HuPrime^{®} liver cancer LI6664 subcutaneous xenograft model in BALB/c nude mice after treatment with sorafenib and AST-3424.
Figure 7 shows the tumor growth curves of the HuPrime^{®} lung cancer LU5173 subcutaneous xenograft model in BALB/c nude mice after treatment with AST-3424.
Figure 8 shows the tumor growth curves of the HuPrime^{®} lung cancer LU5161 subcutaneous xenograft model in BALB/c nude mice after treatment with AST-3424.
Figure 9 shows the tumor growth curves of the HuPrime^{®} pancreatic cancer PA1280 subcutaneous xenograft model in BALB/c nude mice after treatment with AST-3424.
Figure 10 shows the tumor growth curves of the HuPrime^{®} pancreatic cancer PA2637 subcutaneous xenograft model in BALB/c nude mice after treatment with AST-3424.
Figure 11 shows the tumor growth curves of the HuPrime^{®} pancreatic cancer PA9451 subcutaneous xenograft model in BALB/c nude mice after treatment with AST-3424.
Figure 12 shows the tumor growth curves of the HuPrime^{®} pancreatic cancer PA1170 subcutaneous xenograft mouse animal model after treatment with AST-3424.
Figure 13 shows the single-dose toxicokinetic parameters of AST-3424 and AST-2660 under two dosing regimens (D1+D8/QD×5).
Figure 14 shows the repeated-administration toxicological characteristics of AST-3424 under two administration regimens (D1+D8/QD×5), where ALT = Alanine Aminotransferase; AST = Aspartate Aminotransferase; MTD = Maximum Tolerated Dose; HNSTD = Highest Non-Severely Toxic Dose; RBC = Red Blood Cells; #EOS = Absolute Eosinophil Count; %EOS = % Eosinophils; #RET = Absolute Reticulocyte Count; %RETIC = % Reticulocytes; #NEUT= Absolute Neutrophil Count; #LYMPH = Absolute Lymphocyte Count; %LYMPH = % Lymphocytes; WBC = White Blood Cells; HGB = Hemoglobin; HCT = Hematocrit.

*: At a dose of 0.4 mg/kg/day, one male and one female animal were moribund on Day 10 and Day 12, respectively. Consequently, the dose of 0.4 mg/kg was reduced to 0.3 mg/kg starting from the second cycle of administration (i.e., Day 22 of the trial).

### Examples

### Universal instructions

To demonstrate that the new high-frequency, low-dose dosing regimen has acceptable safety and potentially better efficacy compared with the original regimen, the applicant conducted non-clinical animal experiments.

All animal experiments were performed by a professional CRO, and the experimental procedures complied with the relevant requirements of animal experiment ethics.

The relevant tumor tissues used in the experiment all showed moderate to high expression of the AKR1C3 enzyme after detection.

### I. Non-clinical efficacy experiments

The in vivo efficacy of AST-3424 was evaluated in the following models: human liver cancer (HepG2-GFP) orthotopic CDX model in nude mice, HuPrime^{®} liver cancer LI6643 subcutaneous xenograft PDX model, HuPrime^{®} liver cancer LI6664 subcutaneous xenograft PDX model; human lung cancer (H460-GFP) subcutaneous CDX model in nude mice, HuPrime^{®} lung cancer LU5173 subcutaneous xenograft PDX model, HuPrime^{®} lung cancer LU5161 subcutaneous xenograft PDX model; HuPrime^{®} pancreatic cancer PA1280 subcutaneous xenograft PDX model, HuPrime^{®} pancreatic cancer PA2637 subcutaneous xenograft PDX model, HuPrime^{®} pancreatic cancer PA9451 subcutaneous xenograft PDX model, and HuPrime^{®} pancreatic cancer PA1170 subcutaneous xenograft PDX model.

For the experimental procedures and relevant evaluation indicators of the aforementioned solid tumor animal efficacy models, please refer to Examples 7, 8, and 9 in Patent Application 1, namely PCT/US2016/062114, with Publication No. WO2017/087428 (corresponding to Chinese Application CN201680044608.1, with Publication No. CN108290911B), Examples 3 and 4 in Patent Application 2, namely PCT/CN2021/078115, with Publication No. WO2022178821, and Research Literature 1. The aforementioned non-clinical animal efficacy models (PDX) mentioned in the present application are all similar to those in the aforementioned literatures, and the specific operation procedures thereof are not described herein again.

### 1.1 Efficacy analysis and comparison of AST-3424 in liver cancer models

### 1.1.1 Human liver cancer (HepG2-GFP) orthotopic CDX model in nude mice (QW×2 regimen)

In the human liver cancer (HepG2-GFP) orthotopic CDX model in nude mice, the relative tumor growth inhibition rate (TGI) was compared among the following groups: the negative control group (normal saline), the positive control group (sorafenib 30 mg/kg, QD×35, intragastric administration), the AST 3424 low-dose group (1.25 mg/kg, QW×2, drug free for one week, QW×2, totaling four administrations, I.V.), the medium-dose group (2.5 mg/kg, QW×2, drug free for one week, QW×2, totaling 4 administrations , I.V.), and the high-dose group (5 mg/kg, QW×2, drug free for one week, QW×2, totaling four administrations, I.V). The tumor burden of mice was non-invasively collected twice a week using the Fluor Vivo Model 100 imaging system, which showed that AST-3424 had a strong inhibitory effect on the orthotopic model of human liver cancer in mice, with a significant dose-effect relationship. The data from this experiment correspond to the data of the HepG2 model in Example 3-1 of the aforementioned Patent Application 2 (see Table 5 and Figures 4A, 4B of this application). For AST-3424 in this efficacy model, the dosing regimen was administration once per week for 2 consecutive weeks. This regimen is a similar regimen to that used in the Phase I clinical trials of AST-3424 (OBI-3424) conducted in China and the United States, herein referred to as the "original regimen" for short.

### 1.1.2 HuPrime^{®} liver cancer LI6643 subcutaneous xenograft PDX model (QD×5 and QW regimens)

AST-3424 exhibited a significant tumor growth inhibitory effect on the HuPrime^{®} liver cancer LI6643 subcutaneous xenograft model in BALB/c nude mice. This experiment evaluated efficacy and safety by comparing the relative tumor growth inhibition rate TGI (%), animal body weight changes and mortality among the vehicle negative control group (7.5% anhydrous ethanol+7.5% polyoxyethylene (35) castor oil + 85% glucose injection D5W (pH 7.4)), sorafenib (30 mg/kg) treatment group, AST-3424 (0.3 mg/kg) treatment group, AST-3424 (1 mg/kg) treatment group, and AST-3424 (1.25 mg/kg) treatment group.

The modeled BALB/c nude mice were randomly divided into groups (6 mice per group). Except for the sorafenib group (30 mg/kg) administered via intragastric administration (QD×21, totaling 21 administrations), the control group (7.5% anhydrous ethanol+7.5% polyoxyethylene (35) castor oil + 85% glucose injection D5W, pH 7.4; QW×3, totaling 3 administrations) and AST-3424 low-, medium-, and high-dose groups (0.3 mg/kg, 1 mg/kg, 1.25 mg/kg) were all administered via tail vein injection. For the AST-3424 low-dose and medium-dose groups, the drug was administered QD for 5 consecutive days, followed by a 16-day drug-free period, and then another 5 consecutive days of administration, totaling 10 administrations. For the AST-3424 high-dose group, the drug was administered QW for 2 consecutive weeks, followed by a 1-week drug-free period, and then another 2 consecutive weeks of administration, totaling 4 administrations.

The results showed that on Day 21 after the first administration, the relative tumor growth inhibition rate TGI (%) for the AST-3424 low-dose (QD) group (0.3 mg/kg) was 60.16%, showing a statistical difference (P < 0.05) compared to the control group. The TGI (%) for the medium-dose (1 mg/kg) and high-dose (1.25 mg/kg) groups were 86.32% and 71.86%, respectively, both showing statistical differences (P < 0.001 and P < 0.01, respectively) compared to the control group. Furthermore, no difference (P > 0.05) in TGI was shown between the AST-3424 medium-dose (QD) group and high-dose (QW) group, although the TGI value for the medium-dose (QD) group was greater than the TGI value for the high-dose (QW) group. For details, please see the tumor growth curves of the HuPrime^{®} liver cancer LI6643 subcutaneous xenograft model in BALB/c nude mice after treatment with sorafenib and AST-3424 in Figure 4.

### 1.1.3 HuPrime^{®} liver cancer LI6664 subcutaneous xenograft PDX model (QD×5 and QW regimens)

AST-3424 exhibited a significant tumor growth inhibitory effect in the HuPrime^{®} liver cancer LI6664 subcutaneous xenograft model in BALB/c nude mice. The experiment evaluated the efficacy and safety of AST-3424 by comparing the relative tumor growth inhibition rate TGI (%), animal body weight changes and mortality among the vehicle control group, the positive control group, and the AST-3424 administration group.

The modeled BALB/c nude mice were randomly divided into groups (6 mice per group). Except for the sorafenib group (30 mg/kg, QD×21, totaling 21 administrations) administered via intragastric administration, the vehicle control group (7.5% anhydrous g ethanol+7.5% polyoxyethylene (35) castor oil + 85% glucose injection D5W, pH 7.4; QW×3, totaling 3 administrations), and AST-3424 low-, medium-, and high-dose groups (0.3 mg/kg, 1 mg/kg, 1.25 mg/kg) were all administered via tail vein injection. Among them, for the AST-3424 low-dose and medium-dose groups, the drug was administered once daily for 5 consecutive days, followed by a 16-day drug-free period, and then another 5 consecutive days of administration, totaling 10 administrations; for the high-dose group, the drug was administered once weekly for 2 consecutive weeks, followed by a 1-week drug-free period, and then another 2 consecutive weeks of administration, totaling 4 administrations.

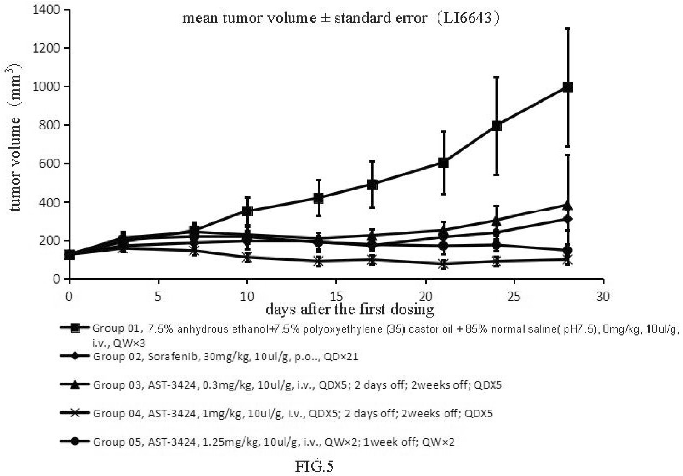

The results showed that on Day 28 after the first administration, for the AST-3424 low-dose (QD) and high-dose (QW) groups, the relative tumor growth inhibition rates TGI (%) were 45.85% and 52.42%, respectively, showing no statistical difference (P > 0.05) compared to the control group; the AST-3424 medium-dose group (QD) showed a TGI (%) of 65.05%, showing a statistical difference (P < 0.05) compared to the control group. There was no difference in TGI between the medium-dose (QD) and high-dose (QW) groups of AST-3424 (P > 0.05), although the TGI value of the medium-dose group (QD) was higher than that of the high-dose group (QW). Safety results showed that except for only a few mice in the control group and all treatment groups being euthanized due to tumor volume exceeding 3000 mm³, no mice were euthanized due to severe body weight loss. All AST-3424 treatment groups were well tolerated during the experiment. For details, please see the tumor growth curves of HuPrime^{®} liver cancer LI6664 subcutaneous xenografts in BALB/c nude mice after treatment with sorafenib and AST-3424 in Figure 5.

The above study results demonstrated that AST-3424 exhibits a significant inhibitory effect on human liver cancer models. Moreover, under the premise of favorable tolerability, the new dosing regimen (QD×5) can increase the total administered dose during the treatment cycle, thereby yielding a superior therapeutic effect compared to the original regimen (QW×2) (as evidenced by higher TGI values after the QD administration period).

### 1.2 Efficacy analysis and comparison of AST-3424 in lung cancer models

### 1.2.1 Human lung cancer (H460-GFP) orthotopic CDX model in nude mice (QW×2 regimen)

In the study investigating the effect of AST-3424 on the human lung cancer (H460-GFP) subcutaneous CDX model in nude mice, the relative tumor growth inhibition (TGI) was compared among the negative control group, the Taxol group (15 mg/kg, BIW×4, I.V.), the AST-3424 low-dose group (0.625 mg/kg, QW×2; 1-week off; QW×2, I.V.), the medium-dose AST-3424 group (1.25 mg/kg, QW×2; 1 week off; QW×2, I.V.), and the AST-3424 high-dose group (2.5 mg/kg, QW×2; 1 week off; QW×2, I.V.). Images of tumor burden in mice were non-invasively collected twice a week by a Fluor Vivo Model 100 imaging system, and the mean value of tumor area in each group was calculated based on imaging analysis results. The experimental results demonstrated that the TGI values for the low, medium, and high-dose AST-3424 groups were 60.2%, 67.2%, and 88%, respectively; 15 mg/kg Taxol showed a TGI of 64%. The anti-tumor efficacy of AST-3424 is comparable to that of Taxol. Both Taxol and AST-3424 at the tested doses exhibited significant inhibitory effects on the human lung cancer (H460-GFP) mouse subcutaneous model, showing statistically significant differences (all P<0.01) compared to the control group. A dose-effect relationship was shown between the AST-3424 high-dose group and the AST-3424 low-dose group. The data from this experiment are the data for the H460 model in Example 3-1 of the aforementioned Patent Application 2 (see Table 5 and Figure 5 of this application). For AST-3424 in this efficacy model, its dosing regimen was once weekly for 3 or 6 consecutive administrations. This regimen is similar to the dosing regimen in the Phase I clinical trials of AST-3424 (OBI-3424) conducted in China and the United States, referred to herein as the "original regimen".

### 1.2.2 HuPrime^{®} lung cancer LU5173 subcutaneous xenograft PDX model (QD×5 regimen)

The antitumor effect of AST-3424 in the HuPrime^{®} lung cancer LU5173 subcutaneous xenograft model in NOD/SCID nude mice was evaluated in Project E4354-B2116.

The LU5173 is a HuPrime^{®} xenograft model established from a lung cancer (small cell lung carcinoma) tumor of a female patient.

The modeled NOD/SCID mice were randomly divided into the following groups (6 mice per group): the vehicle control group, the ifosfamide positive control group (6.0 mg/kg, QD×5/week, for 2 weeks), and the AST-3424 group (1 mg/kg, administered for 5 consecutive days, followed by a 16-day drug-free period, then another 5 consecutive days of administration, totaling 10 administrations over 2 cycles). Efficacy was evaluated based on the relative tumor growth inhibition (TGI, %), and safety was evaluated based on animal body weight changes and mortality.

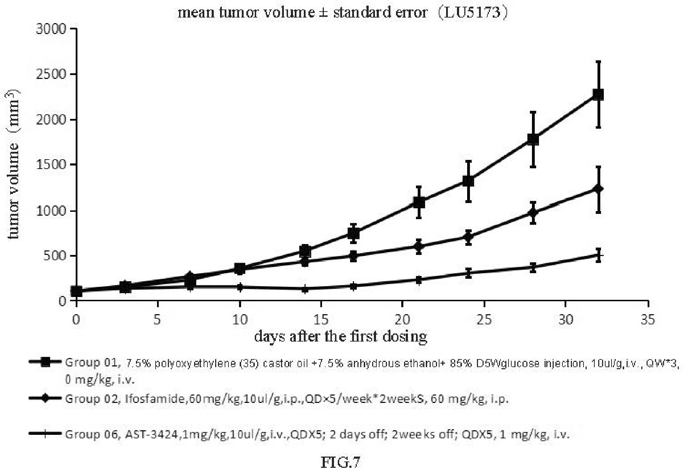

No animal deaths occurred in the AST-3424 group, and it was well tolerated during the treatment period. On day 32 after the first administration, the mean tumor volumes of mice in the vehicle control group, ifosfamide group, and AST-3424 group were 2276.68, 1229.15, and 503.91 mm³, respectively; the TGI (%) in the ifosfamide group was 44.45%, showing no statistical difference (p > 0.05) compared to the control group. The TGI (%) in the AST-3424 group was 78.56%, showing a statistically significant difference (P < 0.001) compared to the control group. For details, please refer to the tumor growth curves of the HuPrime^{®} lung cancer LU5173 subcutaneous xenograft model in BALB/c nude mice after treatment with AST-3424 in Figure 7.

### 1.2.3 HuPrime^{®} lung cancer LU5161 subcutaneous xenograft PDX model (QD×5 regimen)

The anti-tumor activity of AST-3424 in the HuPrime^{®} lung cancer LU5161 subcutaneous xenograft model in BALB/c nude mice was evaluated in Project E4354-B2131.

LU5161 is a HuPrime^{®} xenograft model established from a lung tumor (small cell lung cancer) of a female patient.

The modeled BALB/c nude mice were randomly divided into the following groups (6 mice per group): the negative control group (glucose injection), the ifosfamide positive control group (60 mg/kg, QD×5/week for 2 weeks), and the AST-3424 group (1 mg/kg, administered consecutively for 5 days, followed by a 16-day drug-free period, then another 5 consecutive days of administration, totaling 10 administrations over 2 cycles). Efficacy was evaluated based on the relative tumor growth inhibition rate (TGI, %), and safety was evaluated based on animal body weight changes and mortality.

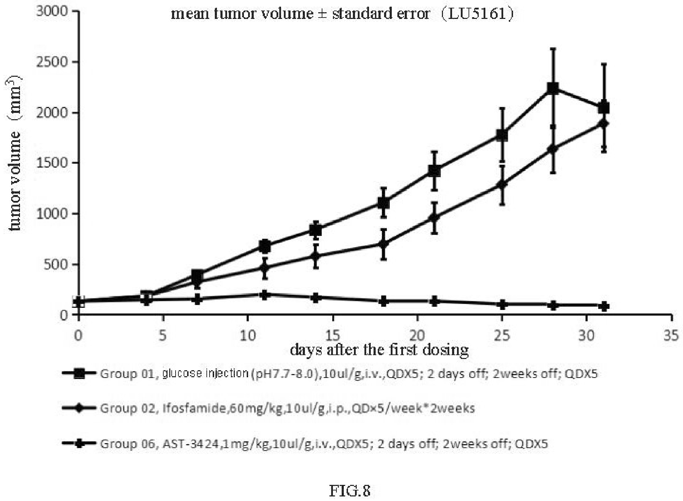

No animal deaths occurred in the AST-3424 group, and it was well tolerated during the treatment period. On day 29 after the first administration, the mean tumor volumes of the negative control group, ifosfamide group, and AST-3424 group were 2238.97, 1636.39, and 94.42 mm³, respectively. The TGI (%) in the ifosfamide group was 27.39%, showing no statistical difference (p > 0.05) compared to the control group. In contrast, the TGI (%) in the AST-3424 group was 95.62%, showing a statistically significant difference (P < 0.001) compared to the control group, and the tumor in one mouse was cleared. For details, please refer to the tumor growth curves of the HuPrime^{®} lung cancer LU5161 subcutaneous xenograft model in BALB/c nude mice after treatment with AST-3424 in Figure 8.

The above study results demonstrated that AST-3424 exhibits a significant inhibitory effect on human lung cancer models. Under the premise of favorable tolerability, the new dosing regimen (QD×5) can increase the total dose administered within the treatment cycle, thereby yielding a superior therapeutic effect compared to the original regimen (QW×2) (as evidenced by a higher TGI value following administration).

### 1.3 Efficacy analysis and comparison in the AST-3424 pancreatic cancer model

### 1.3.1 HuPrime^{®} pancreatic cancer PA1280 subcutaneous xenograft model (QW×3 regimen)

The antitumor effect of AST-3424 in the HuPrime^{®} pancreatic cancer PA1280 subcutaneous xenograft model in BALB/c nude mice was evaluated in Project E4354-B1802.

PA1280 is a HuPrime^{®} xenograft model established from a pancreatic cancer tumor of a female patient.

The modeled BALB/c nude mice were randomly divided into the following groups (6 mice per group): the AST-3424 (2.5 mg/kg) group and the D5W (pH 7.4) vehicle control group. The drug was administered via tail vein injection once weekly for a total of three weeks, followed by one week of observation. Efficacy was evaluated based on the relative tumor growth inhibition rate (TGI%), and safety was evaluated based on animal body weight changes and mortality.

No animal deaths occurred in the AST-3424 (2.5 mg/kg) treatment group, no apparent drug toxicity was observed, and it was well tolerated during the treatment.

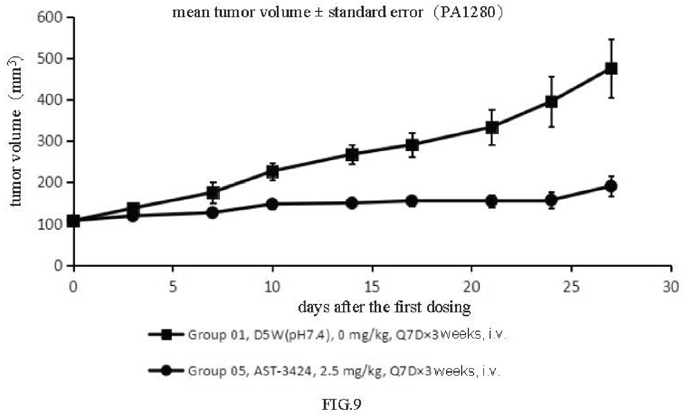

The mean tumor volume of mice in the vehicle control group was 475.07 mm³ on the 7^{th} day after the end of the 3-week administration cycle (Day 27). The mean tumor volume of the test drug AST-3424 (2.5 mg/kg) treatment group was 190.34 mm³ on the 7^{th} day after the end of the 3-week administration cycle (Day 27), showing a statistically significant difference (P < 0.05) compared to the control group, with a relative tumor growth inhibition rate TGI(%) of 60%. For the tumor growth curves of each group, please refer to the tumor growth curves of the HuPrime^{®} pancreatic cancer PA1280 subcutaneous xenograft model in BALB/c nude mice after treatment with AST-3424 in Figure 9.

The experimental results demonstrated that AST-3424 at a dose of 2.5 mg/kg exhibited a statistically significant inhibitory effect on the tumor growth of the HuPrime^{®} pancreatic cancer PA1280. The tumor-bearing mice tolerated AST-3424 well at the tested dose.

### 1.3.2 HuPrime^{®} pancreatic cancer PA2637 subcutaneous xenograft model (QD×5 regimen)

The antitumor effect of AST-3424 in the HuPrime^{®} pancreatic cancer PA2637 subcutaneous xenograft model in NOD.SCID mice was evaluated in Project E4354-B2131.

PA2637 is a HuPrime^{®} xenograft model established from a pancreatic cancer tumor of a patient.

The modeled mice were randomly divided into the following groups (6 mice per group): the negative control group (glucose injection), the ifosfamide positive control group (60 mg/kg, QD×5/week for 2 weeks), and the AST-3424 group (1 mg/kg, administered consecutively for 5 days, followed by a 16-day drug-free period, then another 5 consecutive days of administration, totaling 10 administrations over 2 cycles). Efficacy was evaluated based on the relative tumor growth inhibition rate (TGI, %), and safety was evaluated based on animal body weight changes and mortality.

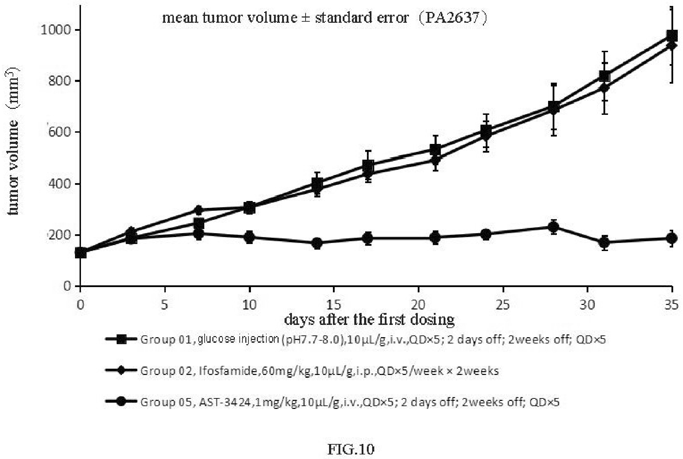

No animal deaths occurred in the AST-3424 group, no apparent drug toxicity was observed, and it was well tolerated during the treatment. On Day 36 after the first administration, the mean tumor volumes in the vehicle control group, the AST-3424 group, and the ifosfamide group were 977.46 mm³, 186.08 mm³, and 938.33 mm³, respectively. The TGI (%) for the ifosfamide group was 2.74%, showing no statistical difference compared to the control group. In contrast, the TGI (%) for the AST-3424 group was 80.78%, showing a statistically significant difference (P < 0.001) compared to the control group, and the tumor in one mouse was cleared. For the tumor growth curves of each group, please refer to the tumor growth curves of the HuPrime^{®} pancreatic cancer PA2637 subcutaneous xenograft model in BALB/c nude mice after treatment with AST-3424 in Figure 10.

### 1.3.3 HuPrime^{®} pancreatic cancer PA9451 subcutaneous xenograft model (QD×5 and QW regimens)

The antitumor effect of AST-3424 in the HuPrime^{®} pancreatic cancer PA9451 subcutaneous xenograft model in BALB/c nude mice was evaluated in Project E4354-B2138.

PA9451 is a HuPrime^{®} xenograft model established from a pancreatic cancer tumor of a patient.

The modeled mice were randomly divided into the following groups (6 mice per group): vehicle control group (glucose injection); the olaparib positive control group (50 mg/kg, QD×30); the AST-3424 low-dose group (0.5 mg/kg, administered consecutively for 5 days, followed by a 16-day drug-free period, then another 5 consecutive days of administration, totaling 10 administrations over 2 cycles), the medium-dose group (1 mg/kg, administered for 5 consecutive days, followed by a 16-day drug-free period, then another 5 consecutive days of administration, totaling 10 administrations over 2 cycles), and the high-dose group (2 mg/kg, administered once weekly for 2 weeks). Efficacy was evaluated based on the relative tumor growth inhibition rate TGI (%), and safety was evaluated based on animal body weight changes and mortality.

No animal deaths occurred in the AST-3424 group, no apparent drug toxicity was observed, and they were well tolerated during the treatment.

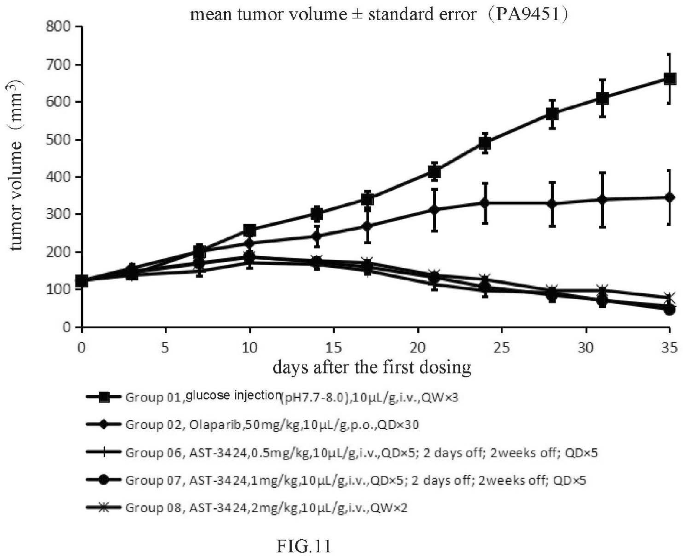

On Day 36 after the first administration, the mean tumor volumes of mice in the vehicle control group and the olaparib group were 661.61 mm³ and 344.84 mm³, respectively. The TGI (%) for the olaparib group was 47.88%, showing a statistically significant difference (P < 0.001) compared to the control group. For the AST-3424 low-dose (QD), medium-dose (QD), and high-dose (QW) groups, the mean tumor volumes were 54.83 mm³, 46.05 mm³, and 76.78 mm³, respectively, and the TGI (%) were 91.77%, 92.99%, and 88.53%, respectively, all showing statistically significant differences (P<0.001) compared to the control group. There were no statistically significant differences among the AST-3424 groups. For the tumor growth curves of each group, please refer to the tumor growth curves of the HuPrime^{®} pancreatic cancer PA9451 subcutaneous xenograft model in BALB/c nude mice after treatment with AST-3424 in Figure 11.

The above study results demonstrated that AST-3424 exhibits a significant inhibitory effect on human pancreatic cancer models. Moreover, under the premise of favorable tolerability, the new dosing regimen (QD×5) can increase the total administered dose during the treatment cycle, thereby yielding a superior therapeutic effect compared to the original regimen (QW×2) (as evidenced by higher TGI values after the QD administration period).

### 1.3.4 HuPrime^{®} pancreatic cancer PA1170 subcutaneous xenograft model (QD×5 and QW regimens)

The antitumor effect of AST-3424 in the HuPrime^{®} pancreatic cancer PA1170 subcutaneous xenograft model in BALB/c nude mice was evaluated in Project E4354-B2135.

PA1170 is a HuPrime^{®} xenograft model established from a pancreatic cancer tumor of a patient.

The modeled mice were randomly divided into the following groups (6 mice per group): the vehicle control group (10% anhydrous ethanol + 10% polyoxyethylene (35) castor oil + 80% glucose injection D5W (pH 7.4)); the olaparib positive control group (50 mg/kg, QD×30); the AST-3424 administration group (1 mg/kg, administered for 5 consecutive days, followed by a 16-day drug-free period, then another 5 consecutive days of administration, totaling 10 administrations over 2 cycles); and the AST-3424 administration group (2 mg/kg, administered once weekly for 2 weeks). Efficacy was evaluated based on the relative tumor growth inhibition rate TGI (%), and safety was evaluated based on animal body weight changes and mortality.

No animal deaths occurred in the AST-3424 group, no apparent drug toxicity was observed, and they were well tolerated during the treatment.

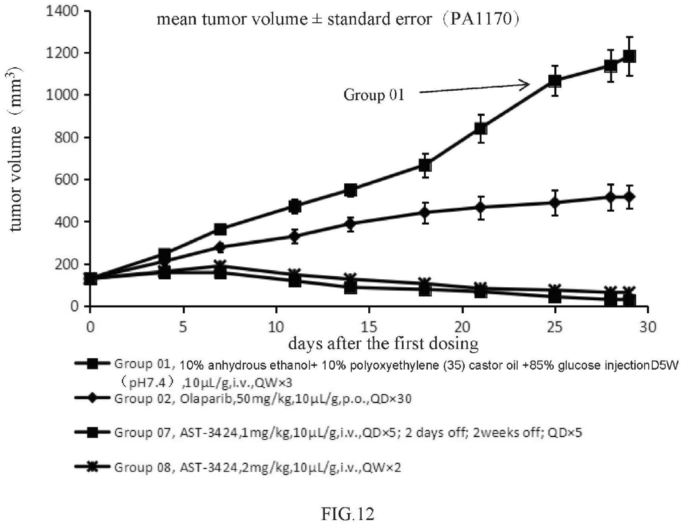

On Day 30 after the first administration, the mean tumor volumes of mice in the vehicle control group and the olaparib group were 1182.66 mm³ and 517.75 mm³, respectively. The TGI (%) for the olaparib group was 57.66%, showing no statistically significant difference (P > 0.05) compared to the control group. For the AST-3424 (1 mg/kg, QD) and (2 mg/kg, QW) groups, the mean tumor volumes were 29.50 mm³ and 64.50 mm³, respectively, and the TGI (%) were 97.60% and 94.83%, respectively, showing statistically significant differences (P<0.05) compared to the control group. There was no statistically significant difference among the AST-3424 groups. For the tumor growth curves of each group, please refer to the tumor growth curves of the HuPrime^{®} pancreatic cancer PA1170 subcutaneous xenograft model in BALB/c nude mice after treatment with AST-3424 in Figure 12.

The above study results demonstrated that AST-3424 exhibits a significant inhibitory effect on human pancreatic cancer models. Moreover, under the premise of favorable tolerability, the new dosing regimen (QD×5) can increase the total administered dose during the treatment cycle, thereby yielding a superior therapeutic effect compared to the original regimen (QW×2) (as evidenced by higher TGI values after the QD administration period).

### 1.4 Efficacy and preliminary safety analysis and comparison of the new regimen (QD×5) versus the old regimen (QW×2) for AST-3424 in the treatment of various solid tumors: liver cancer, lung cancer, and pancreatic cancer

In summary, AST-3424 exhibits significant inhibitory effects on human liver cancer, lung cancer, and pancreatic cancer models. Moreover, under the premise of favorable tolerability, the new dosing regimen (QD×5) can increase the total administered dose during the treatment cycle, thereby yielding a superior therapeutic effect compared to the original regimen (QW×2) (as evidenced by higher TGI values after the QD administration period).

### II. Non-clinical Safety Experiment - Toxicokinetics Study

### 2.1 Toxicokinetics (D1+D8/21 Days)

The toxicokinetics (TK) of AST-3424 were evaluated in Sprague-Dawley (SD) rats (8 /dose/sex). AST-3424 at doses of 3, 10, and 30 mg/kg was administered by a single injection on D1, D8, D22, and D29, respectively. AST-3424 and AST-2660 were rapidly cleared, with mean half-lives of 0.145-0.373 h and 0.589-1.00 h, respectively. The exposure (Cmax and AUCₗₐₛₜ) of AST-3424 increased linearly but not in a dose-proportional manner with increasing doses. Compared to Day 1, AST-3424 showed moderate accumulation on Day 29, while the exposure of AST-2660 decreased.

The toxicokinetics of AST-3424 were also evaluated in cynomolgus monkeys. AST-3424 at doses of 0.1, 0.3, and 0.6 mg/kg was administered by a single 30-minute intravenous infusion on D1, D8, D22, and D29, respectively. On Day 1 and Day 29, Cmax (maximum plasma concentration) and AUClast (area under the concentrationtime curve from the start of administration to the time of the last measurable sample collection) increased linearly but were not in a dose-proportional manner with increasing doses in cynomolgus monkeys. The mean half-lives of AST-3424 and AST-2660 were 0.231-0.592 h and 0.919-1.26 h, respectively, and were independent of dose, time, or sex. The volume of distribution of AST-3424 was equal to or greater than the extracellular fluid volume (223-676 ml/kg), with a clearance (Cl) close to the hepatic plasma flow (705-1090 ml/h/kg). On Day 29 compared to Day 1, the ratios of Cmax and AUClast ranged from 0.90 to 1.21, and no accumulation of AST-3424 was observed. The exposure to AST-2660 was 27.2% and 11.0% of the AST-3424 exposure observed on Day 1 and Day 29, respectively.

### 2.2 Toxicokinetics (QD×5/21 Days)

This study evaluated the toxicokinetic characteristics of cynomolgus monkeys after the administration of AST-3424 injection once daily for 5 consecutive days by a 30-minute intravenous infusion, followed by a 16-day drug-free period as one cycle, with repeated administration for 5 cycles. The experiment included a placebo control group (0 mg/kg), a low-dose AST-3424 injection group (0.1 mg/kg), a medium-dose group (0.2 mg/kg), and a high-dose group (0.4/0.3 mg/kg; the dose was 0.4 mg/kg in the first cycle, and was adjusted to 0.3 mg/kg from the second cycle onwards due to the euthanasia of 2 animals in a moribund state).

After repeated administration for 5 cycles in both female and male cynomolgus monkeys, the exposure (measured by AUC(0-t)) of the prototype drug AST-3424 and its metabolite AST-2660 increased with the increase of the administered dose, with no significant gender differences or exposure accumulation observed. The plasma concentration of the prototype drug AST-3424 reached the peak immediately after the end of administration (Tmax, time to peak concentration), with a half-life (T_{1/2}) of approximately 0.20-0.78 hours. The metabolite AST-2660 reached the peak (Tmax) approximately 0.73-1.25 hours after the start of administration, with a half-life (T_{1/2}) of approximately 0.82-1.23 hours. The exposure of the metabolite AST-2660 accounted for 5.25% to 12.27% of the exposure of the prototype drug.

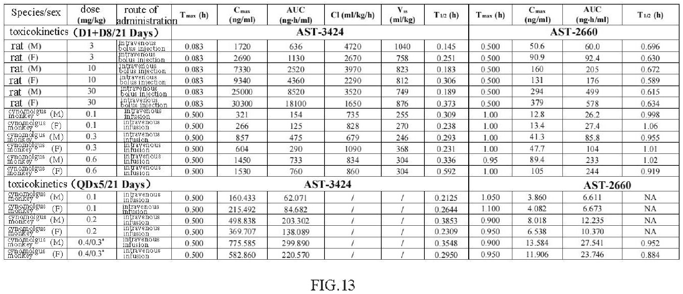

Figure 13 shows the single-dose toxicokinetic parameters of AST-3424 and AST-2660 for the above two dosing regimens.

The experiments for the above two dosing regimens indicate that there is little difference in the toxicokinetics between AST-3424 and its metabolite AST-2660. It can be considered that the new daily dosing regimen exhibits toxicokinetic parameters similar to the previous weekly dosing regimen, with minimal differences. Alternatively, the daily dosing regimen with a lower dose results in reduced exposure to its metabolite AST-2660, which may potentially lead to fewer or milder adverse reactions in human trials.

### III. Non-clinical safety experiments-repeated-administration toxicology study

### 3.1 Repeated-administration toxicity study (D1+D8/21 Days)

In a Good Laboratory Practice (GLP)-compliant repeated-administration toxicity study in Sprague-Dawley (SD) rats, AST-3424 was administered via intravenous bolus injection at doses of 0, 3, 10, and 30 mg/kg on Days 1, 8, 22, and 29 according to the clinical dose regimen for two cycles. Rats developed test article-related, dose-dependent lesions at the injection sites and the distal ends of the tails. At the 10 mg/kg dose, there were mild and reversible changes in hematology and serum biochemistry parameters. The no-observed-adverse-effect level (NOAEL) in rats was determined to be 3 mg/kg, and the dose at which 10% of animals showed severe toxic reactions (STD10) was 30 mg/kg.

In a GLP repeated-administration toxicity study in cynomolgus monkeys, AS-3424 was intravenously infused to cynomolgus monkeys at doses of 0, 0.1, 0.3, and 0.6 mg/kg over 30 minutes on Days 1, 8, 22, and 29. After multiple administrations in cynomolgus monkeys, the observed most prominent toxic reaction induced by AST-3424 was diarrhea and vomiting, with intestinal necrosis/hyperplasia identified in pathological examinations. Meanwhile, AST-3424 exerted mild but reversible effects on erythrocyte-related hematological parameters. Ultimately, the highest non-severe toxic dose (HNSTD) in monkeys was determined to be 0.6 mg/kg.

### 3.2 Repeated-administration toxicity study (QD×5/21 Days)

In a GLP repeated-administration toxicity study in cynomolgus monkeys, AS-3424 was intravenously infused to cynomolgus monkeys at doses of 0, 0.1, 0.2, or 0.4/0.3 mg/kg over 30 minutes on Days 1-5, Days 22-26, Days 43-47, Days 64-68, and Days 85-89. After multiple administrationsin in cynomolgus monkeys, AST-3424 was observed to primarily induce soft stools and watery stools. Pathological examination revealed slight to mild mucosal atrophy in the duodenum and jejunum, and slight to moderate mucosal atrophy in the caecum and colon. AST-3424 had a slight but reversible effect on hematological parameters related to red blood cells and white blood cells. The study determined that the HNSTD of AST-3424 in cynomolgus monkeys under the experimental conditions was 0.3 mg/kg, and the toxic target organs were the duodenum, jejunum, colon, and cecum.

During the trials, a decrease in #EOS (absolute eosinophil count), %EOS (% eosinophils), #RET (absolute reticulocyte count), and %RETIC (% reticulocytes) was primarily observed in the ≥0.1 mg/kg dose groups; a decrease in #NEUT, #LYMPH (lymphocyte absolute count), and %LYMPH (% lymphocyte) was also seen in the ≥ 0.2 mg/kg dose groups; and a decrease in WBC (white blood cell), HGB (hemoglobin), and HCT (hematocrit) was also seen in the 0.4/0.3 mg/kg dose group. Among them, the reduction in eosinophils was correlated with the reduction in the percentage of eosinophils in the bone marrow smears at the end of the administration period, and the decrease in lymphocytes was correlated with the reduction of lymphocytes in the thymic cortex under microscopic examination, which were considered to be secondary stress responses. All the above hematological changes were recoverable during the recovery period.

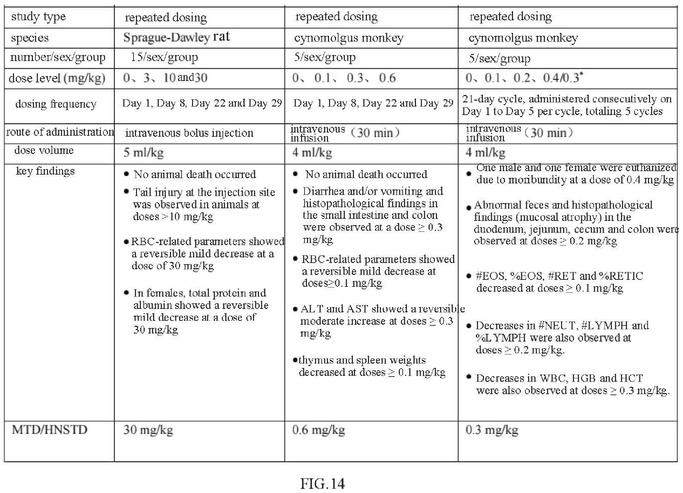

Figure 14 shows the toxicological characteristics of repeated administration of AST-3424 under the above two dosing regimens.

The experiments involving the above two dosing regimens indicate that the toxicological studies of AST-3424 reveal that toxic reactions of both the daily dosing regimen and the weekly dosing regimen were concentrated in the blood system and gastrointestinal tract, with similar adverse reactions, and no new adverse reactions were induced by the high-frequency dosing regimen. The high-frequency daily dosing regimen resulted in a reduction of the HNSTD in cynomolgus monkeys to 0.3 mg/kg, which suggests that the final dose for the daily dosing regimen in subsequent human clinical trials may be lower than the 6 mg/m² for the weekly dosing regimen.

### Animal model experiment summary

Compared to the former dosing regimen adopted in the Phase I clinical trial of AST-3424 (OBI-3424), which was a 21-day cycle with administration on Day 1 and Day 8, the new dosing regimen has been preliminarily validated for efficacy and preliminary safety and tolerability through various solid tumor PDX animal models. The new dosing regimen has further verified its safety and tolerability at specific doses through toxicokinetic studies and repeated-dose toxicology studies in cynomolgus monkeys. The new dosing regimen has further verified the safety and tolerability of a series of new specific dosing regimens through human clinical trial safety modeling predictions. In summary, the new dosing regimen compared to the former dosing regimen, may have advantages in terms of efficacy and safety for the further human clinical trials of the drug in the next stage: better efficacy and milder adverse reactions.

Since the chemical structure of TFX05-01 is similar to that of AST-3424, and it similarly produces AST-2660 upon activation by AKR1C3 to further exert tumor inhibitory effects, the applicant infers that this compound has metabolic properties and behaviors similar to those of AST-3424 in the human body. Therefore, it can be predicted that TFX05-01 will have animal experiment/clinical trial data similar to those of AST-3424. Compared to its dosing regimen of a 21-day cycle, with administration on Day 1 and Day 8, adopted in its ongoing Phase I clinical trial, the new dosing regimen designed in the present application may have advantages in efficacy and safety: better efficacy and milder adverse reactions.

All patent applications cited in the present application are incorporated into the description by reference in their entirety.

Non-patent literatures (academic journal papers, academic conference papers, etc.) cited in the present application are as follows:
Reference 1: Meng F, Li WF, Jung D, et al. A novel selective AKR1C3-activated prodrug AST-3424/OBI-3424 exhibits broad anti-tumor activity. Am J Cancer Res. 2021;11(7):3645-3659;
Reference 2: Evans K, Duan J, Pritchard T, et al. OBI-3424, a Novel AKR1C3-Activated Prodrug, Exhibits Potent Efficacy against Preclinical Models of T-ALL. Clin Cancer Res. 2019;25(14):4493-4503. doi:10.1158/1078-0432.CCR-19-0551;
Reference 3: Wang Y, Liu Y, Zhou C, et al. An AKR1C3-specific prodrug with potent anti-tumor activities against T-ALL. Leuk Lymphoma. 2020;61(7):1660-1668. doi:10.1080/10428194.2020.1728746;
Reference 4: He P, Wang C, Wang Y, et al. A Novel AKR1C3 Specific Prodrug TH3424 With Potent Antitumor Activity in Liver Cancer [retracted in: Clin Pharmacol Ther. 2021 Jul;110(1):262]. Clin Pharmacol Ther. 202, 110(1):229-237. doi:10.1002/cpt.2171;
Reference 5:
   Study poster: Tsimberidou, Apostolia & Verschraegen, Claire & Hsu, Pei & Pearce, Tillman. (2022). Safety, pharmacokinetics, and clinical activity of OBI-3424, an AKR1C3-activated prodrug, in patients with advanced or metastatic solid tumors: A phase 1 dose-escalation study. Journal of Clinical Oncology. 40. 3030-3030.10.1200/JCO.2022.40.16_suppl.3030, the poster can be downloaded from the official website of OBI Pharma Inc at https://www.obipharma.com/news/news-2022/poster-presentations-at-the-2022-asco-annual-meeting-for-adagloxad-simolenin-obi-999-and-obi-3424/;
   and Study article: Tsimberidou AM, Verschraegen CF, Wesolowski R, Shia CS, Hsu P, Pearce TE. Phase 1 dose-escalation study evaluating the safety, pharmacokinetics, and clinical activity of OBI-3424 in patients with advanced or metastatic solid tumors. Br J Cancer. 2023;129(2):266-274. doi:10.1038/s41416-023-02280-4;
Reference 6: Zhang Y, Qin S, Chao J, Luo Y, Sun Y and Duan J (2022) The In-Vitro Antitumor Effects of AST-3424 Monotherapy and Combination Therapy With Oxaliplatin or 5-Fluorouracil in Primary Liver Cancer. Front. Oncol. 12:885139. doi: 10.3389/fonc.2022.885139;
Reference 7: Charles Z Ding, Zhe Cai, Wei Sha. Preclinical evaluation of TFX05-01, a selective AKR1C3- targeted prodrug for solid tumor [abstract]. In: Proceedings of the American Association for Cancer Research Annual Meeting 2022; 2022 Apr 8-13. Philadelphia (PA): AACR; Cancer Res 2022;82(12_Suppl): Abstract nr 5691. and the corresponding AACR2022 Poster.

Unless otherwise specified, all English abbreviations in the present application shall be construed in accordance with their definitions in pharmaceutical and medical textbooks.

## Claims

1. A dosing regimen for treating human tumors with an AKR1C3-activated DNA alkylating agent prodrug compound that is metabolized to AST-2660, in monotherapy or combination therapy, **characterized in that** an interval between two consecutive administrations within one treatment cycle is 8-144 hours, preferably 12-72 hours, and a number of administrations within one treatment cycle is not less than 3; the tumors are preferably malignant tumors, and the AKR1C3 enzyme-activated prodrug is selected from compounds of formulae (1)-(6): 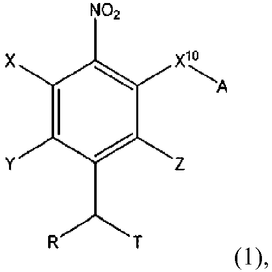 wherein X, Y, Z, R, A and X¹⁰ are as defined in the claims of Patent Application PCT/US2016/021581, with Publication No. WO2016145092A1 (corresponding to Chinese Application No. 2016800150788, with Publication No. CN107530556A); T is 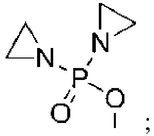 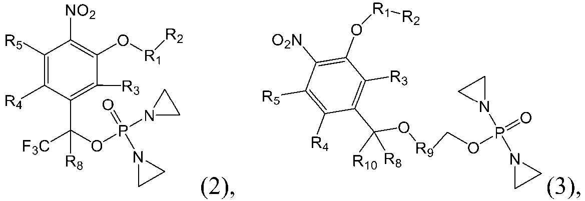 wherein R₁, R₂, R₃, R₄, R₅, R₈, R₉ and R₁₀ are as defined in the claims of Patent Application PCT/CN2020/089692, with Publication No. WO2020228685A9 (corresponding to Chinese Application No. 2020800358890, with Publication No. CN113853379A); 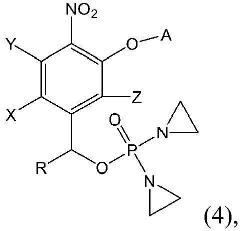 wherein:
A is substituted or unsubstituted C₆-C₁₀ aryl, biaryl, or substituted biaryl, 5- to 15-membered heteroaryl, or -N=CR¹R², wherein a substituent for substitution is selected from the group consisting of: halogen, -CN, -NO₂, -O-(CH₂)-O-, -CO₂H and salts thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, - SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5- to 7-membered heterocyclic ring;
wherein the alkyl and aryl are each substituted by 1-3 halogens or 1-3 C₁-C₆ alkyls;
R¹ and R² are each independently phenyl or methyl;
X, Y and Z are each independently hydrogen or halogen;
R is hydrogen or C₁-C₆ alkyl or halogen-substituted alkyl. 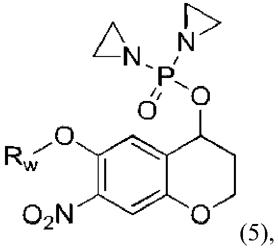
wherein Rw is as defined in the claims of Patent Application PCT/CN2020/120281, with Publication No. WO2021068952A1 (corresponding to Chinese Application No. 202080071652.8, with Publication No. CN114555574A); 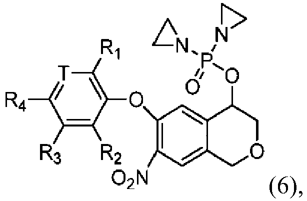
wherein R₁, R₂, R₃, R₄ and T are as defined in the claims of Patent Application PCT/CN2021/118597, with Publication No. WO2022057838A1.

2. A dosing regimen for treating human tumors with AST-3424, in monotherapy or combination therapy, **characterized in that** an interval between two consecutive administrations within one treatment cycle is 8-144 hours, preferably 12-72 hours, and a number of administrations within one treatment cycle is not less than 3, and the tumors are preferably malignant tumors.

3. The dosing regimen according to claim 1 or 2, **characterized in that** the interval between two consecutive administrations within one treatment cycle is 8, 12, 24, 48, 72, 96, 120, or 144 hours, and the tumors include solid tumors, which are selected from hepatocellular carcinoma, colorectal cancer, gastric cancer, intrahepatic cholangiocarcinoma, pancreatic cancer, submandibular gland carcinoma, prostate cancer, breast cancer, and melanoma, and hematological tumors, which are selected from acute lymphoblastic leukemia, preferably acute T-lymphoblastic leukemia and acute B-lymphoblastic leukemia.

4. The dosing regimen according to claim 3, **characterized in that** the interval between two consecutive administrations in one treatment cycle is 12, 24, 48, or 72 hours, and the administration route is intravenous injection, preferably intravenous drip, with a dose of 1-8 mg/m² per administration.

5. The dosing regimen according to claim 3, **characterized in that** one treatment cycle is 21 days, specifically selected from the following regimens:
administering once daily on Days 1, 2, and 3 (totaling 3 administrations) respectively, with no administration on the remaining 18 days;
administering once daily on Days 1, 2, 3, and 4 (totaling 4 administrations) respectively, with no administration on the remaining 17 days;
administering once daily on Days 1, 2, 3, 4, and 5 (totaling 5 administrations) respectively, with no administration on the remaining 16 days;
administering once daily on Days 1, 2, 3, 4, 5, and 6 (totaling 6 administrations) respectively, with no administration on the remaining 15 days;
administering once daily on Days 1, 2, 3, 4, 5, 6, and 7 (totaling 7 administrations) respectively, with no administration on the remaining 14 days;
administering once daily on Days 1, 3, and 5 (totaling 3 administrations) respectively, with no administration on the remaining 18 days;
administering once daily on Days 1, 3, 5, and 7 (totaling 4 administrations) respectively, with no administration on the remaining 17 days;
administering once daily on Days 1, 3, 5, 7, and 9 (totaling 5 administrations) respectively, with no administration on the remaining 16 days;
administering once daily on Days 1, 4, and 7 (totaling 3 administrations) respectively, with no administration on the remaining 18 days;
administering once daily on Days 1, 5, and 9 (totaling 3 administrations) respectively, with no administration on the remaining 18 days.

6. The dosing regimen according to claim 3, **characterized in that** one treatment cycle is 14 days, specifically selected from the following regimens:
administering once daily on Days 1, 2, and 3 (totaling 3 administrations) respectively, with no administration on the remaining 11 days;
administering once daily on Days 1, 2, 3, and 4 (totaling 4 administrations) respectively, with no administration on the remaining 10 days;
administering once daily on Days 1, 2, 3, 4, and 5 (totaling 5 administrations) respectively, with no administration on the remaining 9 days;
administering once daily on Days 1, 2, 3, 4, 5, and 6 (totaling 6 administrations) respectively, with no administration on the remaining 8 days;
administering once daily on Days 1, 2, 3, 4, 5, 6 and 7 (totaling 7 administrations) respectively, with no administration on the remaining 7 days;
administering once daily on Days 1, 3, and 5 (totaling 3 administrations) respectively, with no administration on the remaining 11 days;
administering once daily on Days 1, 3, 5, and 7 (totaling 4 administrations) respectively, with no administration on the remaining 10 days;
administering once daily on Days 1, 3, 5, 7, and 9 (totaling 5 administrations) respectively, with no administration on the remaining 9 days;
administering once daily on Days 1, 4, and 7 (totaling 3 administrations) respectively, with no administration on the remaining 11 days.

7. The dosing regimen according to claim 3, **characterized in that** one treatment cycle is 28 days, specifically selected from the following regimens:
administering once daily on Days 1, 2, and 3 (totaling 3 administrations) respectively, with no administration on the remaining 25 days;
administering once daily on Days 1, 2, 3, and 4 (totaling 4 administrations) respectively, with no administration on the remaining 24 days;
administering once daily on Days 1, 2, 3, 4, and 5 (totaling 5 administrations) respectively, with no administration on the remaining 23 days;
administering once daily on Days 1, 2, 3, 4, 5, and 6 (totaling 6 administrations) respectively, with no administration on the remaining 22 days;
administering once daily on Days 1, 2, 3, 4, 5, 6, and 7 (totaling 7 administrations) respectively, with no administration on the remaining 21 days;
administering once daily on Days 1, 2, 3, 4, 5, 6, 7 and 8 (totaling 8 administrations) respectively, with no administration on the remaining 20 days;
administering once daily on Days 1, 2, 3, 4, 5, 6, 7, 8 and 9 (totaling 9 administrations) respectively, with no administration on the remaining 19 days;
administering once daily on Days 1, 2, 3, 4, 5, 6, 7, 8, 9 and 10 (totaling 10 administrations) respectively, with no administration on the remaining 18 days;
administering once daily on Days 1, 3, and 5 (totaling 3 administrations) respectively, with no administration on the remaining 25 days;
administering once daily on Days 1, 3, 5, and 7 (totaling 4 administrations) respectively, with no administration on the remaining 24 days;
administering once daily on Days 1, 3, 5, 7, and 9 (totaling 5 administrations) respectively, with no administration on the remaining 23 days;
administering once daily on Days 1, 4, and 7 (totaling 3 administrations) respectively, with no administration on the remaining 25 days;
administering once daily on Days 1, 4, 7 and 10 (totaling 4 administrations) respectively, with no administration on the remaining 24 days;
administering once daily on Days 1, 4, 7, 10 and 13 (totaling 5 administrations) respectively, with no administration on the remaining 23 days;
administering once daily on Days 1, 5, and 9 (totaling 3 administrations) respectively, with no administration on the remaining 25 days;
administering once daily on Days 1, 5, 9 and 13 (totaling 4 administrations) respectively, with no administration on the remaining 24 days.

8. Pharmaceutical use of an AKR1C3-activated DNA alkylating agent prodrug compound that is metabolized to AST-2660, in the manufacture of a medicament for treating human tumors, in monotherapy or combination therapy, **characterized in that** an interval between two consecutive administrations within one treatment cycle is 8-144 hours, preferably 12-72 hours, and the number of administrations within one treatment cycle is not less than 3; the tumors are preferably malignant tumors, and the AKR1C3 enzyme-activated prodrug is selected from compounds of formulae (1)-(6): 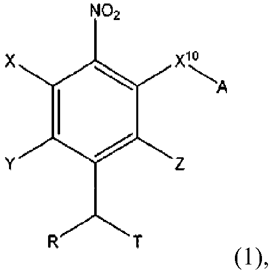 wherein X, Y, Z, R, A and X¹⁰ are as defined in the claims of Patent Application PCT/US2016/021581, with Publication No. WO2016145092A1 (corresponding to Chinese Application No. 2016800150788, with Publication No. CN107530556A); T is 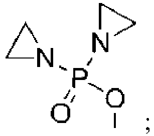 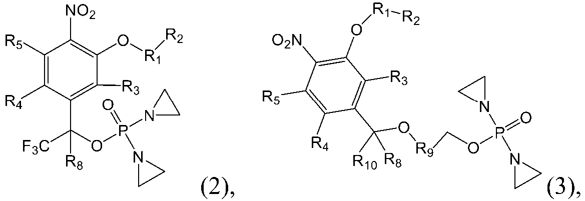 wherein R₁, R₂, R₃, R₄, R₅, R₈, R₉ and R₁₀ are as defined in the claims of Patent Application PCT/CN2020/089692, with Publication No. WO2020228685A9 (corresponding to Chinese Application No. 2020800358890, with Publication No. CN113853379A); 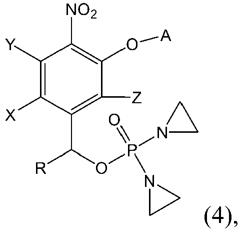 wherein:
A is substituted or unsubstituted C₆-C₁₀ aryl, biaryl, or substituted biaryl, 5-15 membered heteroaryl, or -N=CR¹R², wherein a substituent for substitution is selected from the group consisting of: halogen, -CN, -NO₂, -O-(CH₂)-O-, -CO₂H and salts thereof, -OR¹⁰⁰, -CO₂R¹⁰⁰, -CONR¹⁰¹R¹⁰², -NR¹⁰¹R¹⁰², -NR¹⁰⁰SO₂R¹⁰⁰, -SO₂R¹⁰⁰, - SO₂NR¹⁰¹R¹⁰², C₁-C₆ alkyl, and C₃-C₁₀ heterocyclyl;
wherein R¹⁰⁰, R¹⁰¹ and R¹⁰² are each independently hydrogen, C₁-C₈ alkyl, or C₆-C₁₂ aryl; or R¹⁰¹ and R¹⁰² together with the nitrogen atom to which they are attached form a 5-7 membered heterocyclic ring;
wherein the alkyl and aryl are each substituted by 1-3 halogens or 1-3 C₁-C₆ alkyls;
R¹ and R² are each independently phenyl or methyl;
X, Y and Z are each independently hydrogen or halogen;
R is hydrogen or C₁-C₆ alkyl or halogen-substituted alkyl. 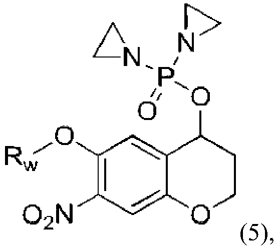
wherein Rw is as defined in the claims of Patent Application PCT/CN2020/120281, with Publication No. WO2021068952A1 (corresponding to Chinese Application No. 202080071652.8, with Publication No. CN114555574A); 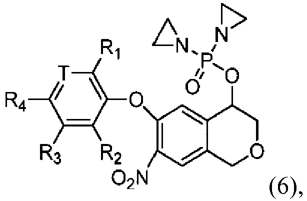
wherein R₁, R₂, R₃, R₄ and T are as defined in the claims of Patent Application PCT/CN2021/118597, with Publication No. WO2022057838A1.

9. Pharmaceutical use of AST-3424 in the manufacture of a medicament for treating human tumors, in monotherapy or combination therapy, **characterized in that** an interval between two consecutive administrations within one treatment cycle is 8-144 hours, preferably 12-72 hours, and the number of administrations within one treatment cycle is not less than 3, and the tumors are preferably malignant tumors.

10. The pharmaceutical use according to claim 8 or 9, **characterized in that** the interval between two consecutive administrations within one treatment cycle is 8, 12, 24, 48, 72, 96, 120, or 144 hours, and the tumors include solid tumors, which are selected from hepatocellular carcinoma, colorectal cancer, gastric cancer, intrahepatic cholangiocarcinoma, pancreatic cancer, submandibular gland carcinoma, prostate cancer, breast cancer, and melanoma, and hematological tumors, which are selected from acute lymphoblastic leukemia, preferably acute T-lymphoblastic leukemia and acute B-lymphoblastic leukemia.

11. The pharmaceutical use according to claim 10, **characterized in that** the interval between two consecutive administrations in one treatment cycle is 12, 24, 48, or 72 hours, and the administration route is intravenous injection, preferably intravenous drip, with a dose of 1-8 mg/m² per administration.

12. The pharmaceutical use according to claim 10, **characterized in that** the treatment cycle is selected from 14 days, 21 days or 28 days,
where when one treatment cycle is 21 days, the dosing regimen is specifically selected from the following:
administering once daily on Days 1, 2, and 3 (totaling 3 administrations) respectively, with no administration on the remaining 18 days;
administering once daily on Days 1, 2, 3, and 4 (totaling 4 administrations) respectively, with no administration on the remaining 17 days;
administering once daily on Days 1, 2, 3, 4, and 5 (totaling 5 administrations) respectively, with no administration on the remaining 16 days;
administering once daily on Days 1, 2, 3, 4, 5, and 6 (totaling 6 administrations) respectively, with no administration on the remaining 15 days;
administering once daily on Days 1, 2, 3, 4, 5, 6, and 7 (totaling 7 administrations) respectively, with no administration on the remaining 14 days;
administering once daily on Days 1, 3, and 5 (totaling 3 administrations) respectively, with no administration on the remaining 18 days;
administering once daily on Days 1, 3, 5, and 7 (totaling 4 administrations) respectively, with no administration on the remaining 17 days;
administering once daily on Days 1, 3, 5, 7, and 9 (totaling 5 administrations) respectively, with no administration on the remaining 16 days;
administering once daily on Days 1, 4, and 7 (totaling 3 administrations) respectively, with no administration on the remaining 18 days;
administering once daily on Days 1, 5, and 9 (totaling 3 administrations) respectively, with no administration on the remaining 18 days;
wherein when one treatment cycle is 14 days, the dosing regimen is specifically selected from the following:
administering once daily on Days 1, 2, and 3 (totaling 3 administrations) respectively, with no administration on the remaining 11 days;
administering once daily on Days 1, 2, 3, and 4 (totaling 4 administrations) respectively, with no administration on the remaining 10 days;
administering once daily on Days 1, 2, 3, 4, and 5 (totaling 5 administrations) respectively, with no administration on the remaining 9 days;
administering once daily on Days 1, 2, 3, 4, 5, and 6 (totaling 6 administrations) respectively, with no administration on the remaining 8 days;
administering once daily on Days 1, 2, 3, 4, 5, 6 and 7 (totaling 7 administrations) respectively, with no administration on the remaining 7 days;
administering once daily on Days 1, 3, and 5 (totaling 3 administrations) respectively, with no administration on the remaining 11 days;
administering once daily on Days 1, 3, 5, and 7 (totaling 4 administrations) respectively, with no administration on the remaining 10 days;
administering once daily on Days 1, 3, 5, 7, and 9 (totaling 5 administrations) respectively, with no administration on the remaining 9 days;
administering once daily on Days 1, 4, and 7 (totaling 3 administrations) respectively, with no administration on the remaining 11 days;
wherein when one treatment cycle is 28 days, the dosing regimen is specifically selected from the following:
administering once daily on Days 1, 2, and 3 (totaling 3 administrations) respectively, with no administration on the remaining 25 days;
administering once daily on Days 1, 2, 3, and 4 (totaling 4 administrations) respectively, with no administration on the remaining 24 days;
administering once daily on Days 1, 2, 3, 4, and 5 (totaling 5 administrations) respectively, with no administration on the remaining 23 days;
administering once daily on Days 1, 2, 3, 4, 5, and 6 (totaling 6 administrations) respectively, with no administration on the remaining 22 days;
administering once daily on Days 1, 2, 3, 4, 5, 6, and 7 (totaling 7 administrations) respectively, with no administration on the remaining 21 days;
administering once daily on Days 1, 2, 3, 4, 5, 6, 7 and 8 (totaling 8 administrations) respectively, with no administration on the remaining 20 days;
administering once daily on Days 1, 2, 3, 4, 5, 6, 7, 8 and 9 (totaling 9 administrations) respectively, with no administration on the remaining 19 days;
administering once daily on Days 1, 2, 3, 4, 5, 6, 7, 8, 9 and 10 (totaling 10 administrations) respectively, with no administration on the remaining 18 days;
administering once daily on Days 1, 3, and 5 (totaling 3 administrations) respectively, with no administration on the remaining 25 days;
administering once daily on Days 1, 3, 5, and 7 (totaling 4 administrations) respectively, with no administration on the remaining 24 days;
administering once daily on Days 1, 3, 5, 7, and 9 (totaling 5 administrations) respectively, with no administration on the remaining 23 days;
administering once daily on Days 1, 4, and 7 (totaling 3 administrations) respectively, with no administration on the remaining 25 days;
administering once daily on Days 1, 4, 7 and 10 (totaling 4 administrations) respectively, with no administration on the remaining 24 days;
administering once daily on Days 1, 4, 7, 10 and 13 (totaling 5 administrations) respectively, with no administration on the remaining 23 days;
administering once daily on Days 1, 5, and 9 (totaling 3 administrations) respectively, with no administration on the remaining 25 days;
administering once daily on Days 1, 5, 9 and 13 (totaling 4 administrations) respectively, with no administration on the remaining 24 days.
